# EUROPEAN PATENT SPECIFICATION

(11) **EP 1 995 245 B1**
(45) Date of publication and mention of the grant of the patent: **02.05.2012**
(21) Application number: 07738761.1
(22) Date of filing: 09.03.2007
(51) Int. Cl.: C07D 303/30, C07D 301/28

(54) **METHOD FOR PRODUCING EPOXY COMPOUND**
VERFAHREN ZUR HERSTELLUNG EINER EPOXYVERBINDUNG
PROCÉDÉ PERMETTANT DE PRODUIRE UN COMPOSÉ EPOXY

(30) Priority: 16.03.2006 JP 2006072574
(43) Date of publication of application: 26.11.2008
(73) Proprietor: Sumitomo Chemical Company, Limited, Tokyo 104-8260 (JP)
(72) Inventor: TANAKA, Shinya, Toyono-gun, Osaka 563-0103 (JP); HIBINO, Hiroaki, Toyonaka-shi, Osaka 561-0802 (JP)
(74) Representative: Vossius & Partner
(86) International application number: PCT/JP2007/055314
(87) International publication number: WO 2007/105809

(56) References cited:
- WO-A1-2005/061473
- JP-A- 61 178 974
- JP-B- 47 032 838
- US-A- 2 943 096
- US-A- 3 221 032

## Description

The present invention relates to a process for producing an epoxy compound.

An epoxy compound represented by the formula (3): 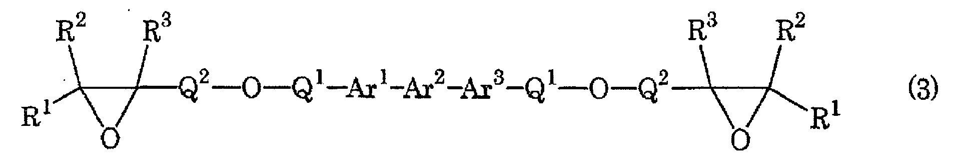 wherein,
Q¹ denotes a single bond or a straight-chain alkylene group having 1 to 9 carbon atoms, in which at least one methylene group composing the straight-chain alkylene group is optionally substituted with an alkyl group having 1 to 8 carbon atoms and -O- or -N(R⁴)- is optionally inserted between the methylene groups, in which R⁴ denotes hydrogen atom or an alkyl group having 1 to 8 carbon atoms;
Q² denotes a straight-chain alkylene group having 1 to 8 carbon atoms, in which at least one methylene group composing the straight-chain alkylene group is optionally substituted with an alkyl group having 1 to 8 carbon atoms;
Ar¹, Ar² and Ar³ are the same or different and each denotes any one of divalent groups represented by the following formulas: 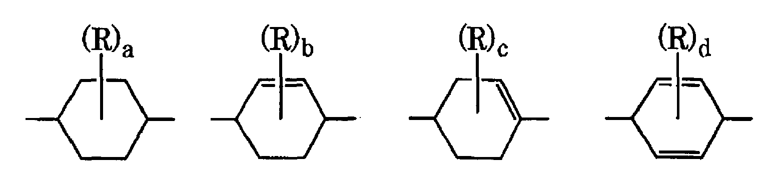 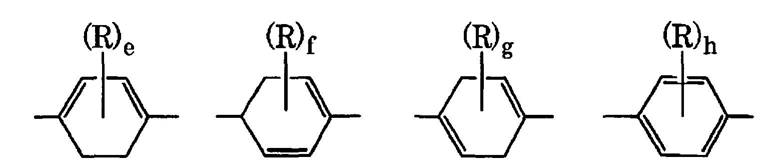 in which R denotes an alkyl group having 1 to 8 carbon atoms, a denotes an integer of 0 to 8, b, e and g denotes an integer of 0 to 6, c denotes an integer of 0 to 7, d and h denote an integer of 0 to 4, and f denotes an integer of 0 to 5, and when more than one R exists in said divalent group, all of R may be the same group or different groups; and
R¹, R² and R³ are the same or different and each denotes hydrogen atom or an alkyl group having 1 to 8 carbon atoms; had a lower melt temperature and can be melt-blended with a curing agent at a curing temperature or lower, as described in EP 1698625 A1. A cured epoxy resin obtained by curing the epoxy compound with the use of a curing agent not only exhibits liquid crystallinity, but also has a high thermal conductivity, so that it is useful as an insulating material which requires a high heat dissipation capacity, such as a printed wiring board.

EP 1698625 A1 discloses a method comprising heating the corresponding dihydroxy compound with epihalohydrin in the presence of an alkali metal hydroxide to react. US-2 943 096 discloses the reaction of polyhydric phenol with epichlorohydrin in the presence of a catalytic amount of an ammonium compound.

However, a further industrial improvement has been required from the point of view of purity of the obtained epoxy compound.

The present invention provides:
<1> a process for producing an epoxy compound represented by the formula (3): 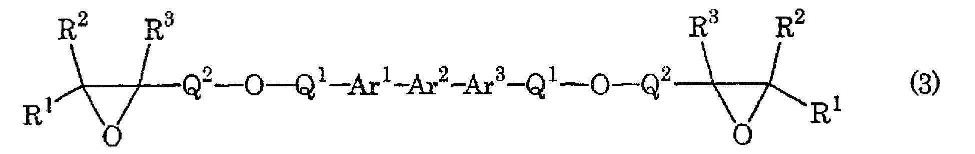 wherein Ar¹, Ar², Ar³, R¹, R², R³, Q¹ and Q² are as defined blow, which comprises reacting a dihydroxy compound represented by the formula (1) :

   HO-Q¹-Ar¹-Ar²-Ar³-Q¹-OH (1)

   wherein,
   Q¹ denotes a single bond or a straight-chain alkylene group having 1 to 9 carbon atoms, in which at least one methylene group composing the straight-chain alkylene group is optionally substituted with an alkyl group having 1 to 8 carbon atoms;
   Ar¹, Ar² and Ar³ are the same or different and each denotes any one of divalent groups represented by the following formulas: 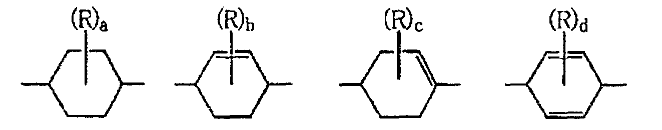 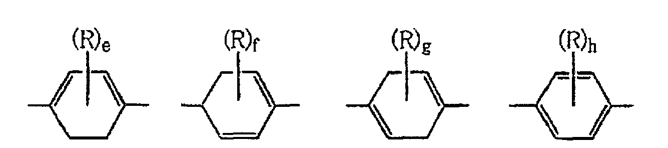 in which R denotes an alkyl group having 1 to 8 carbon atoms, a denotes an integer of 0 to 8, b, e and g denote an integer of 0 to 6, c denotes an integer of 0 to 7, d and h denote an integer of 0 to 4, f denotes an integer of 0 to 5,and when more than one R exists in said divalent group, all of R may be the same group or different groups; with a compound represented by the formula (2): 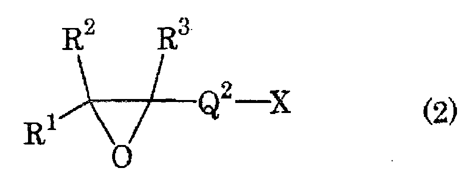 wherein R¹, R² and R³ are the same or different and each denotes a hydrogen atom or an alkyl group having 1 to 8 carbon atoms, Q² denotes a straight-chain alkylene group having 1 to 8 carbon atoms, in which at least one methylene group composing the straight-chain alkylene group is optionally substituted with an alkyl group having 1 to 8 carbon atoms, and X denotes a halogen atom, in the presence of at least one selected from the group consisting of an amine compound and an ammonium salt, and further reacting the obtained reaction mixture and an inorganic base by mixing wherein the inorganic base is added to the reaction mixture after the dihydroxy compound represented by the formula (1) and the compound represented by the formula (2) were reacted and the dihydroxy compound represented by the formula (1) disappeared and 50% or more of a compound obtained by reacting one molecule of the dihydroxy compound represented by the formula (1) and one molecule of the compound represented by the formula (2) disappeared;
<2> the process for producing an epoxy compound according to <1>, wherein an inorganic base is added to the reaction mixture after the dihydroxy compound represented by the formula (1) disappeared and 80% or more of a compound obtained by reacting one molecule of the dihydroxy compound represented by the formula (1) and one molecule of the compound represented by the formula (2) disappeared;
<3> the process for producing an epoxy compound according to <1>, wherein an inorganic base is added to the reaction mixture after the dihydroxy compound represented by the formula (1) disappeared and 90% or more of a compound obtained by reacting one molecule of the dihydroxy compound represented by the formula (1) and one molecule of the compound represented by the formula (2) disappeared;
<4> the process for producing an epoxy compound according to any one of <1> to <3>, wherein the dihydroxy compound represented by the formula (1) and the compound represented by the formula (2) are reacted in the presence of an ammonium salt;
<5> The process for producing an epoxy compound according to any one of <1> to <4>, wherein the amine compound is a tertiary amine;
<6> the process for producing an epoxy compound according to <5>, wherein the tertiary amine is triethylamine;
<7> the process for producing an epoxy compound according to any one of <1> to <4>, wherein the ammonium salt is a quaternary ammonium salt;
<8> the process for producing an epoxy compound according to <7>, wherein the quaternary ammonium salt is a quaternary ammonium halide;
<9> the process for producing an epoxy compound according to <8>, wherein the quaternary ammonium halide is tetraalkylammonium chloride or tetraalkylammonium bromide;
<10> the process for producing an epoxy compound according to any one of according to <1> to <9>, wherein the inorganic base is an alkali metal hydroxide;
<11> the process for producing an epoxy compound according to any one of <1> to <10>, wherein Ar¹ and Ar³ independently denote a group represented by the following formula: 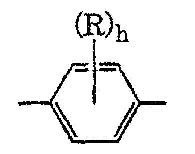 wherein R and h are as defined above;
<12> the process for producing an epoxy compound according to <11>, wherein Ar¹ and Ar³ independently denote a 1,4-phenylene group, 3-methyl-1,4-phenylene group or 3-isopropyl-1,4-phenylene group;
<13> the process for producing an epoxy compound according to any one of <1> to <12>, wherein Ar² is a group represented by the following formula: 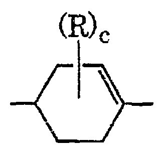 wherein R and c are as defined above;
<14> the process for producing an epoxy compound according to <13>, wherein Ar² is a 1-cyclohexene-1,4-diyl group;
<15> the process for producing an epoxy compound according to any one of <1> to <10>, wherein Ar¹ and Ar³ independently denote a group represented by the following formula: 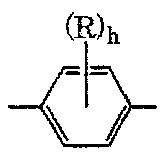 wherein R and h are as defined above, and Ar² denotes a group represented by the following formula: 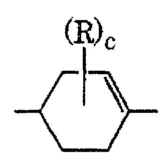 wherein R and c are as defined above; and
<16> the process for producing an epoxy compound according to <17>, wherein Ar¹ and Ar³ independently denote a 1,4-phenylene group, 3-methyl-1,4-phenylene group or 3-isopropyl-1,4-phenylene group, and Ar² denotes a 1-cyclohexene-1,4-diyl group.

In the formula of the dihydroxy compound represented by the formula (1):

HO-Q¹-Ar¹-Ar²-Ar³-Q¹-OH (1)

(hereinafter abbreviated to a dihydroxy compound (1)), Q¹ denotes a single bond or a straight-chain alkylene group having 1 to 9 carbon atoms.

Examples of the straight-chain alkylene group having 1 to 9 carbon atoms include groups formed by bonding 1 to 9 methylene groups linearly, such as methylene, ethylene, trimethylene, tetramethylene, hexamethylene and nonamethylene group. At least one methylene group composing such a straight-chain alkylene group having 1 to 9 carbon atoms is optionally substituted with an alkyl group having 1 to 8 carbon atoms.

Examples of the alkyl group having 1 to 8 carbon atoms include straight-chain, branched chain or cyclic alkyl groups, such as methyl, ethyl, n-propyl, isopropyl, n-butyl, isobutyl, sec-butyl, tert-butyl, n-pentyl, tert-pentyl, n-hexyl, 1-methylpentyl, 1-ethylbutyl, n-heptyl, n-octyl, 2-ethylhexyl, isooctyl, tert-octyl, cyclohexyl and cyclooctyl group.

R⁴ denotes hydrogen atom or an alkyl group of 1 to 8 carbon atom, and examples of the alkyl group having 1 to 8 carbon atoms include the same groups as those described above.

Examples of the alkylene group in which at least one methylene group is substituted with an alkyl group having 1 to 8 carbon atoms or in which -O- or -N(R⁴)- is inserted between the methylene groups include 2-methyltrimethylene, 1,2-dimethylethylene, 3-oxatetramethylene and 3-oxapentamethylene group.

Q¹ is preferably a single bond.

In the formula (1), Ar¹, Ar² and Ar³ are the same or different and each denotes any one of divalent groups represented by the following formulas:

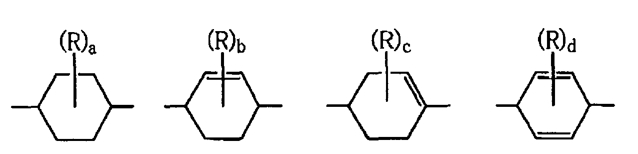

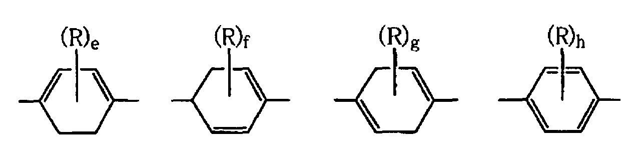

In the formulas of the divalent groups, R denotes an alkyl group having 1 to 8 carbon atoms, a denotes an integer of 0 to 8, b, e and g denote an integer of 0 to 6, c denotes an integer of 0 to 7, d and h denote an integer of 0 to 4, and f denotes an integer of 0 to 5. Examples of the alkyl group having 1 to 8 carbon atoms include the same groups as those described above.

When more than one R exists in said divalent group, all of R may be the same group or different groups.

Examples of the divalent group include cyclohexane-1,4-diyl, 2-cyclohexene-1,4-diyl, 1-cyclohexene-1,4-diyl, 1,4-cyclohexadiene-3,6-diyl, 1,3-cyclohexadiene-1,4-diyl, 1,3-cyclohexanediene-2,5-diyl, 1,4-cyclohexanediene-1,4-diyl, 1,4-phenylene, 2-methylcyclohexane-1,4-diyl, 3-methyl-1,4-phenylene and 3-isopropyl-1,4-phenylene group.

Among them, preferred is a dihydroxy compound (1) in which Ar¹ and Ar³ independently denote a group represented by the following formula: 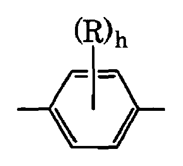 wherein R and h are as defined above, and more preferred is a dihydroxy compound (1) in which Ar¹ and Ar³ independently denote 1,4-phenylene group, 3-methyl-1,4-phenylene group or 3-isopropyl-1,4-phenylene group.

Preferred is a dihydroxy compound (1) in which Ar² denotes the following formula: 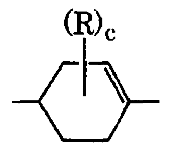 wherein R and c are as defined above, and more preferred is a dihydroxy compound (1) in which Ar² denotes 1-cyclohexene-1,4-diyl group.

Among them, more preferred is a dihydroxy compound (1) in which Ar¹ and Ar³ independently denote a group represented by the following formula: 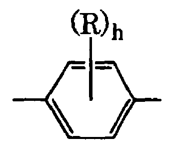 wherein R and h are as defined above, and Ar² denote a group represented by the following formula: 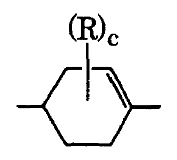 wherein R and c are as defined above, and particularly preferred is a dihydroxy compound (1) in which Ar¹ and Ar³ independently denote 1,4-phenylene group, 3-methyl-1,4-phenylene group or 3-isopropyl-1,4-phenylene group, and Ar² denotes 1-cyclohexene-1,4-diyl group.

Examples of the dihydroxy compound (1) include
1,4-bis(4-hydroxyphenyl)cyclohexane,
1-(2-methyl-4-hydroxyphenyl)-4-(4-hydroxyphenyl)cyclohexane,
1-(3-methyl-4-hydroxyphenyl)-4-(4-hydroxyphenyl)cyclohexane,
1-(3-ethyl-4-hydroxyphenyl)-4-(4-hydroxyphenyl)cyclohexane,
1-(3-n-propyl-4-hydroxyphenyl)-4-(4-hydroxyphenyl)cyclohexane,
1-(3-isopropyl-4-hydroxyphenyl)-4-(4-hydroxyphenyl)cyclohexan e,
1-(3-n-butyl-4-hydroxyphenyl)-4-(4-hydroxyphenyl)cyclohexane,
1-(3-sec-butyl-4-hydroxyphenyl)-4-(4-hydroxyphenyl)cyclohexan e,
1-(3-tert-butyl-4-hydroxyphenyl)-4-(4-hydroxyphenyl)cyclohexa ne,
1-(3-n-pentyl-4-hydroxyphenyl)-4-(4-hydroxyphenyl)cyclohexane,
1-(3-tert-pentyl-4-hydroxyphenyl)-4-(4-hydroxyphenyl)cyclohex ane,
1-(3-n-hexyl-4-hydroxyphenyl)-4-(4-hydroxyphenyl)cyclohexane,
1-{3-(1-methylpentyl)-4-hydroxyphenyl}-4-(4-hydroxyphenyl)cyc lohexane,
1-{3-(1-ethylbutyl)-4-hydroxyphenyl}-4-(4-hydroxyphenyl)cyclo hexane,
1-(3-cyclohexyl-4-hydroxyphenyl)-4-(4-hydroxyphenyl)cyclohexa ne,
1-(3-n-heptyl-4-hydroxyphenyl)-4-(4-hydroxyphenyl)cyclohexane,
1-(3-n-octyl-4-hydroxyphenyl)-4-(4-hydroxyphenyl)cyclohexane,
1-{3-(2-ethylhexyl)-hydroxyphenyl}-4-(4-hydroxyphenyl)cyclohe xane,
1-(3-tert-octyl-4-hydroxyphenyl)-4-(4-hydroxyphenyl)cyclohexa ne,
1-(3-cyclooctyl-4-hydroxyphenyl)-4-(4-hydroxyphenyl)cyclohexa ne,
1,4-bis(4-hydroxyphenyl)-1-cyclohexene,
1-(2-methyl-4-hydroxyphenyl)-4-(4-hydroxyphenyl)-1-cyclohexen e,
1-(3-methyl-4-hydroxyphenyl)-4-(4-hydroxyphenyl)-1-cyclohexen e,
1-(3-ethyl-4-hydroxyphenyl)-4-(4-hydroxyphenyl)-1-cyclohexene,
1-(3-n-propyl-4-hydroxyphenyl)-4-(4-hydroxyphenyl)-1-cyclohex ene,
1-(3-isopropyl-4-hydroxyphenyl)-4-(4-hydroxyphenyl)-1-cyclohe xene,
1-(3-n-butyl-4-hydroxyphenyl)-4-(4-hydroxyphenyl)-1-cyclohexe ne,
1-(3-sec-butyl-4-hydroxyphenyl)-4-(4-hydroxyphenyl)-1-cyclohe xene,
1-(3-tert-butyl-4-hydroxyphenyl)-4-(4-hydroxyphenyl)-1-cycloh exene,
1-(3-n-pentyl-4-hydroxyphenyl)-4-(4-hydroxyphenyl)-1-cyclohex ene,
1-(3-tert-pentyl-4-hydroxyphenyl)-4-(4-hydroxyphenyl)-1-cyclo hexene,
1-(3-n-hexyl-4-hydroxyphenyl)-4-(4-hydroxyphenyl)-1-cyclohexe ne,
1-{3-(1-methylpentyl)-4-hydroxyphenyl}-4-(4-hydroxyphenyl)-1-cyclohexene,
1-{3-(1-ethylbutyl)-4-hydroxyphenyl}-4-(4-hydroxyphenyl)-1-cy clohexene,
1-(3-cyclohexyl-4-hydroxyphenyl)-4-(4-hydroxyphenyl)-1-cycloh exene,
1-(3-n-heptyl-4-hydroxyphenyl)-4-(4-hydroxyphenyl)-1-cyclohex ene,
1-(3-n-octyl-4-hydroxyphenyl)-4-(4-hydroxyphenyl)-1-cyclohexe ne,
1-(3-(2-ethylhexyl)-4-hydroxyphenyl)-4-(4-hydroxyphenyl)-1-cy clohexene,
1-(3-tert-octyl-4-hydroxyphenyl)-4-(4-hydroxyphenyl)-1-cycloh exene,
1-(3-cyclooctyl-4-hydroxyphenyl)-4-(4-hydroxyphenyl)-1-cycloh exene,
1,4-bis(4-hydroxyphenyl)-2-cyclohexene,
1-(2-methyl-4-hydroxyphenyl)-4-(4-hydroxyphenyl)-2-cyclohexen e,
1-(3-methyl-4-hydroxyphenyl)-4-(4-hydroxyphenyl)-2-cyclohexen e,
1-(2-ethyl-4-hydroxyphenyl)-4-(4-hydroxyphenyl)-2-cyclohexene,
1-(3-ethyl-4-hydroxyphenyl)-4-(4-hydroxyphenyl)-2-cyclohexene,
1-(3-n-propyl-4-hydroxyphenyl)-4-(4-hydroxyphenyl)-2-cyclohex ene,
1-(3-isopropyl-4-hydroxyphenyl)-4-(4-hydroxyphenyl)-2-cyclohe xene,
1-(3-n-butyl-4-hydroxyphenyl)-4-(4-hydroxyphenyl)-2-cyclohexe ne,
1-(3-sec-butyl-4-hydroxyphenyl)-4-(4-hydroxyphenyl)-2-cyclohe xene,
1-(3-tert-butyl-4-hydroxyphenyl)-4-(4-hydroxyphenyl)-2-cycloh exene,
1-(3-n-pentyl-4-hydroxyphenyl)-4-(4-hydroxyphenyl)-2-cyclohex ene,
1-(3-tert-pentyl-4-hydroxyphenyl)-4-(4-hydroxyphenyl)-2-cyclo hexene,
1-(3-n-hexyl-4-hydroxyphenyl)-4-(4-hydroxyphenyl)-2-cyclohexe ne,
1-{3-(1-methylpentyl)-4-hydroxyphenyl}-4-(4-hydroxyphenyl)-2-cyclohexene,
1-{3-(1-ethylbutyl)-4-hydroxyphenyl}-4-(4-hydroxyphenyl)-2-cy clohexene,
1-(3-cyclohexyl-4-hydroxyphenyl)-4-(4-hydroxyphenyl)-2-cycloh exene,
1-(3-n-heptyl-4-hydroxyphenyl)-4-(4-hydroxyphenyl)-2-cyclohex ene,
1-(3-n-octyl-4-hydroxyphenyl)-4-(4-hydroxyphenyl)-2-cyclohexe ne,
1-{3-(2-ethylhexyl)-4-hydroxyphenyl}-4-(4-hydroxyphenyl)-2-cy clohexene,
1-(3-tert-octyl-4-hydroxyphenyl)-4-(4-hydroxyphenyl)-2-cycloh exene,
1-(3-cyclooctyl-4-hydroxyphenyl)-4-(4-hydroxyphenyl)-2-cycloh exene,
1,4-bis(4-hydroxyphenyl)-2,5-cyclohexadiene,
1-(2-methyl-4-hydroxyphenyl)-4-(4-hydroxyphenyl)-2,5-cyclohex adiene,
1-(3-methyl-4-hydroxyphenyl)-4-(4-hydroxyphenyl)-2,5-cyclohex adiene,
1-(3-ethyl-4-hydroxyphenyl)-4-(4-hydroxyphenyl)-2,5-cyclohexa diene,
1-(3-n-propyl-4-hydroxyphenyl)-4-(4-hydroxyphenyl)-2,5-cycloh exadiene,
1-(3-isopropyl-4-hydroxyphenyl)-4-(4-hydroxyphenyl)-2,5-cyclo hexadiene,
1-(3-n-butyl-4-hydroxyphenyl)-4-(4-hydroxyphenyl)-2,5-cyclohe xadiene,
1-(3-sec-butyl-4-hydroxyphenyl)-4-(4-hydroxyphenyl)-2,5-cyclo hexadiene,
1-(3-tert-butyl-4-hydroxyphenyl)-4-(4-hydroxyphenyl)-2,5-cycl ohexadiene,
1-(3-n-pentyl-4-hydroxyphenyl)-4-(4-hydroxyphenyl)-2,5-cycloh exadiene,
1-(3-tert-pentyl-4-hydroxyphenyl)-4-(4-hydroxyphenyl)-2,5-cyc lohexadiene,
1-(3-n-hexyl-4-hydroxyphenyl)-4-(4-hydroxyphenyl)-2,5-cyclohe xadiene,
1-{3-(1-methylpentyl)-4-hydroxyphenyl}-4-(4-hydroxyphenyl)-2, 5-cyclohexadiene,
1-{3-(1-ethylbutyl)-4-hydroxyphenyl}-4-(4-hydroxyphenyl)-2,5-cyclohexadiene,
1-(3-cyclohexyl-4-hydroxyphenyl)-4-(4-hydroxyphenyl)-2,5-cycl ohexadiene,
1-(3-n-heptyl-4-hydroxyphenyl)-4-(4-hydroxyphenyl)-2,5-cycloh exadiene,
1-(3-n-octyl-4-hydroxyphenyl)-4-(4-hydroxyphenyl)-2,5-cyclohe xadiene,
1-{3-(2-ethylhexyl)-4-hydroxyphenyl}-4-(4-hydroxyphenyl)-2,5-cyclohexadiene,
1-(3-tert-octyl-4-hydroxyphenyl)-4-(4-hydroxyphenyl)-2,5-cycl ohexadiene,
1-(3-cyclooctyl-4-hydroxyphenyl)-4-(4-hydroxyphenyl)-2,5-cycl ohexadiene,
1,4-bis(4-hydroxyphenyl)-1,5-cyclohexadiene,
1-(2-methyl-4-hydroxyphenyl)-4-(4-hydroxyphenyl)-1,5-cyclohex adiene,
1-(3-methyl-4-hydroxyphenyl)-4-(4-hydroxyphenyl)-1,5-cyclohex adiene,
1-(3-ethyl-4-hydroxyphenyl)-4-(4-hydroxyphenyl)-1,5-cyclohexa diene,
1-(3-n-propyl-4-hydroxyphenyl)-4-(4-hydroxyphenyl)-1,5-cycloh exadiene,
1-(3-isopropyl-4-hydroxyphenyl)-4-(4-hydroxyphenyl)-1,5-cyclo hexadiene,
1-(3-n-butyl-4-hydroxyphenyl)-4-(4-hydroxyphenyl)-1,5-cyclohe xadiene,
1-(3-sec-butyl-4-hydroxyphenyl)-4-(4-hydroxyphenyl)-1,5-cyclo hexadiene,
1-(3-tert-butyl-4-hydroxyphenyl)-4-(4-hydroxyphenyl)-1,5-cycl ohexadiene,
1-(3-n-pentyl-4-hydroxyphenyl)-4-(4-hydroxyphenyl)-1,5-cycloh exadiene,
1-(3-tert-pentyl-4-hydroxyphenyl)-4-(4-hydroxyphenyl)-1,5-cyc lohexadiene,
1-(3-n-hexyl-4-hydroxyphenyl)-4-(4-hydroxyphenyl)-1,5-cyclohe xadiene,
1-{3-(1-methylpentyl)-4-hydroxyphenyl}-4-(4-hydroxyphenyl)-1, 5-cyclohexadiene,
1-{3-(1-ethylbutyl)-4-hydroxyphenyl}-4-(4-hydroxyphenyl)-1,5-cyclohexadiene,
1-(3-cyclohexyl-4-hydroxyphenyl)-4-(4-hydroxyphenyl)-1,5-cycl ohexadiene,
1-(3-n-heptyl-4-hydroxyphenyl)-4-(4-hydroxyphenyl)-1,5-cycloh exadiene,
1-(3-n-octyl-4-hydroxyphenyl)-4-(4-hydroxyphenyl)-1,5-cyclohe xadiene,
1-{3-(2-ethylhexyl)-4-hydroxyphenyl}-4-(4-hydroxyphenyl)-1,5-cyclohexadiene,
1-(3-tert-octyl-4-hydroxyphenyl)-4-(4-hydroxyphenyl)-1,5-cycl ohexadiene,
1-(3-cyclooctyl-4-hydroxyphenyl)-4-(4-hydroxyphenyl)-1,5-cycl ohexadiene,
1,4-bis(4-hydroxyphenyl)-1,4-cyclohexadiene,
1-(2-methyl-4-hydroxyphenyl)-4-(4-hydroxyphenyl)-1,4-cyclohex adiene,
1-(3-methyl-4-hydroxyphenyl)-4-(4-hydroxyphenyl)-1,4-cyclohex adiene,
1-(3-ethyl-4-hydroxyphenyl)-4-(4-hydroxyphenyl)-1,4-cyclohexa diene,
1-(3-n-propyl-4-hydroxyphenyl)-4-(4-hydroxyphenyl)-1,4-cycloh exadiene,
1-(3-isopropyl-4-hydroxyphenyl)-4-(4-hydroxyphenyl)-1,4-cyclo hexadiene,
1-(3-n-butyl-4-hydroxyphenyl)-4-(4-hydroxyphenyl)-1,4-cyclohe xadiene,
1-(3-sec-butyl-4-hydroxyphenyl)-4-(4-hydroxyphenyl)-1,4-cyclo hexadiene,
1-(3-tert-butyl-4-hydroxyphenyl)-4-(4-hydroxyphenyl)-1,4-cycl ohexadiene,
1-(3-n-pentyl-4-hydroxyphenyl)-4-(4-hydroxyphenyl)-1,4-cycloh exadiene,
1-(3-tert-pentyl-4-hydroxyphenyl)-4-(4-hydroxyphenyl)-1,4-cyc lohexadiene,
1-(3-n-hexyl-4-hydroxyphenyl)-4-(4-hydroxyphenyl)-1,4-cyclohe xadiene,
1-{3-(1-methylpentyl)-4-hydroxyphenyl}-4-(4-hydroxyphenyl)-1, 4-cyclohexadiene,
1-{3-(1-ethylbutyl)-4-hydroxyphenyl}-4-(4-hydroxyphenyl)-1,4-cyclohexadiene,
1-(3-cyclohexyl-4-hydroxyphenyl)-4-(4-hydroxyphenyl)-1,4-cycl ohexadiene,
1-(3-n-heptyl-4-hydroxyphenyl)-4-(4-hydroxyphenyl)-1,4-cycloh exadiene,
1-(3-n-octyl-4-hydroxyphenyl)-4-(4-hydroxyphenyl)-1,4-cyclohe xadiene,
1-{3-(2-ethylhexyl)-hydroxyphenyl}-4-(4-hydroxyphenyl)-1,4-cy clohexadiene,
1-(3-tert-octyl-4-hydroxyphenyl)-4-(4-hydroxyphenyl)-1,4-cycl ohexadiene,
1-(3-cyclooctyl-4-hydroxyphenyl)-4-(4-hydroxyphenyl)-1,4-cycl ohexadiene,
1,4-bis(4-hydroxyphenyl)-1,3-cyclohexadiene,
1-(2-methyl-4-hydroxyphenyl)-4-(4-hydroxyphenyl)-1,3-cyclohex adiene,
1-(3-methyl-4-hydroxyphenyl)-4-(4-hydroxyphenyl)-1,3-cyclohex adiene,
1-(3-ethyl-4-hydroxyphenyl)-4-(4-hydroxyphenyl)-1,3-cyclohexa diene,
1-(3-n-propyl-4-hydroxyphenyl)-4-(4-hydroxyphenyl)-1,3-cycloh exadiene,
1-(3-isopropyl-4-hydroxyphenyl)-4-(4-hydroxyphenyl)-1,3-cyclo hexadiene,
1-(3-n-butyl-4-hydroxyphenyl)-4-(4-hydroxyphenyl)-1,3-cyclohe xadiene,
1-(3-sec-butyl-4-hydroxyphenyl)-4-(4-hydroxyphenyl)-1,3-cyclo hexadiene,
1-(3-tert-butyl-4-hydroxyphenyl)-4-(4-hydroxyphenyl)-1,3-cycl ohexadiene,
1-(3-n-pentyl-4-hydroxyphenyl)-4-(4-hydroxyphenyl)-1,3-cycloh exadiene,
1-(3-tert-pentyl-4-hydroxyphenyl)-4-(4-hydroxyphenyl)-1,3-cyc lohexadiene,
1-(3-n-hexyl-4-hydroxyphenyl)-4-(4-hydroxyphenyl)-1,3-cyclohe xadiene,
1-{3-(1-methylpentyl)-4-hydroxyphenyl}-4-(4-hydroxyphenyl)-1, 3-cyclohexadiene,
1-{3-(1-ethylbutyl)-4-hydroxyphenyl}-4-(4-hydroxyphenyl)-1,3-cyclohexadiene,
1-(3-cyclohexyl-4-hydroxyphenyl)-4-(4-hydroxyphenyl)-1,3-cycl ohexadiene,
1-(3-n-heptyl-4-hydroxyphenyl)-4-(4-hydroxyphenyl)-1,3-cycloh exadiene,
1-(3-n-octyl-4-hydroxyphenyl)-4-(4-hydroxyphenyl)-1,3-cyclohe xadiene,
1-{3-(2-ethylhexyl)-4-hydroxyphenyl}-4-(4-hydroxyphenyl)-1,3-cyclohexadiene,
1-(3-tert-octyl-4-hydroxyphenyl)-4-(4-hydroxyphenyl)-1,3-cycl ohexadiene,
1-(3-cyclooctyl-4-hydroxyphenyl)-4-(4-hydroxyphenyl)-1,3-cycl ohexadiene,
1,4-bis(4-hydroxyphenyl)benzene,
1-(2-methyl-4-hydroxyphenyl)-4-(4-hydroxyphenyl)benzene,
1-(3-methyl-4-hydroxyphenyl)-4-(4-hydroxyphenyl)benzene,
1-(3-ethyl-4-hydroxyphenyl)-4-(4-hydroxyphenyl)benzene,
1-(3-n-propyl-4-hydroxyphenyl)-4-(4-hydroxyphenyl)benzene,
1-(3-isopropyl-4-hydroxyphenyl)-4-(4-hydroxyphenyl)benzene,
1-(3-n-butyl-4-hydroxyphenyl)-4-(4-hydroxyphenyl)benzene,
1-(3-sec-butyl-4-hydroxyphenyl)-4-(4-hydroxyphenyl)benzene,
1-(3-tert-butyl-4-hydroxyphenyl)-4-(4-hydroxyphenyl)benzene,
1-(3-n-pentyl-4-hydroxyphenyl)-4-(4-hydroxyphenyl)benzene,
1-(3-tert-pentyl-4-hydroxyphenyl)-4-(4-hydroxyphenyl)benzene,
1-(3-n-hexyl-4-hydroxyphenyl)-4-(4-hydroxyphenyl)benzene,
1-(3-(1-methylpentyl)-4-hydroxyphenyl)-4-(4-hydroxyphenyl)ben zene,
1-(3-(1-ethylbutyl)-4-hydroxyphenyl)-4-(4-hydroxyphenyl)benze ne,
1-(3-cyclohexyl-4-hydroxyphenyl)-4-(4-hydroxyphenyl)benzene,
1-(3-n-heptyl-4-hydroxyphenyl)-4-(4-hydroxyphenyl)benzene,
1-(3-n-octyl-4-hydroxyphenyl)-4-(4-hydroxyphenyl)benzene,
1-{3-(2-ethylhexyl)-4-hydroxyphenyl}-4-(4-hydroxyphenyl)benze ne,
1-(3-tert-octyl-4-hydroxyphenyl)-4-(4-hydroxyphenyl)benzene,
1-(3-cyclooctyl-4-hydroxyphenyl)-4-(4-hydroxyphenyl)benzene,
1,4-bis{4-(2-hydroxyethoxy)phenyl}cyclohexane,
1-{3-methyl-4-(2-hydroxyethoxy)phenyl}-4-{4-(2-hydroxyethoxy) phenyl}cyclohexane,
1,4-bis{4-(3-hydroxypropoxy)phenyl}cyclohexane,
1-{3-methyl-4-(3-hydroxypropoxy)phenyl}-4-{4-(3-hydroxypropox y)phenyl}cyclohexane,
1,4-bis{4-(4-hydroxybutoxy)phenyl}cyclohexane,
1-{3-methyl-4-(4-hydroxybutoxy)phenyl}-4-{4-(4-hydroxybutoxy) phenyl}cyclohexane,
1,4-bis{4-(5-hydroxypentyloxy)phenyl}cyclohexane,
1-{3-methyl-4-(5-hydroxypentyloxy)phenyl}-4-{4-(5-hydroxypent yloxy)phenyl}cyclohexane,
1,4-bis{4-(6-hydroxyhexyloxy)phenyl}cyclohexane,
1-{3-methyl-4-(6-hydroxyhexyloxy)phenyl}-4-{4-(6-hydroxyhexyl oxy)phenyl}cyclohexane,
1,4-bis{4-(8-hydroxyoctyloxy)phenyl}cyclohexane,
1-{3-methyl-4-(8-hydroxyoctyloxy)phenyl}-4-{4-(8-hydroxyoctyl oxy)phenyl}cyclohexane,
1,4-bis{4-(2-hydroxyethoxy)phenyl}-1-cyclohexene,
1-{3-methyl-4-(2-hydroxyethoxy)phenyl}-4-{4-(2-hydroxyethoxy) phenyl}-1-cyclohexene,
1,4-bis{4-(3-hydroxypropoxy)phenyl}-1-cyclohexene,
1-{3-methyl-4-(3-hydroxypropoxy)phenyl}-4-{4-(3-hydroxypropox y)phenyl}-1-cyclohexene,
1,4-bis{4-(4-hydroxybutoxy)phenyl}-1-cyclohexene,
1-{3-methyl-4-(4-hydroxybutoxy)phenyl}-4-{4-(4-hydroxybutoxy) phenyl}-1-cyclohexene,
1,4-bis{4-(5-hydroxypentyloxy)phenyl}-1-cyclohexene,
1-{3-methyl-4-(5-hydroxypentyloxy)phenyl}-4-{4-(5-hydroxypent yloxy)phenyl}-1-cyclohexene,
1,4-bis{4-(6-hydroxyhexyloxy)phenyl}-1-cyclohexene,
1-{3-methyl-4-(6-hydroxyhexyloxy)phenyl}-4-{4-(6-hydroxyhexyl oxy)phenyl}-1-cyclohexene,
1,4-bis{4-(8-hydroxyoctyloxy)phenyl}-1-cyclohexene,
1-{3-methyl-4-(8-hydroxyoctyloxy)phenyl}-4-{4-(8-hydroxyoctyl oxy)phenyl}-1-cyclohexene,
1,4-bis{4-(2-hydroxyethoxy)phenyl}benzene,
1-{3-methyl-4-(2-hydroxyethoxy)phenyl}-4-{4-(2-hydroxyethoxy) phenyl}benzene, 1,4-bis{4-(3-hydroxypropoxy)phenyl}benzene,
1-{3-methyl-4-(3-hydroxypropoxy)phenyl}-4-{4-(3-hydroxypropox y)phenyl}benzene, 1,4-bis{4-(4-hydroxybutoxy)phenyl}benzene,
1-{3-methyl-4-(4-hydroxybutoxy)phenyl}-4-{4-(4-hydroxybutoxy) phenyl}benzene, 1,4-bis{4-(5-hydroxypentyloxy)phenyl}benzene,
1-{3-methyl-4-(5-hydroxypentyloxy)phenyl}-4-{4-(5-hydroxypent yloxy)phenyl}benzene,
1,4-bis{4-(6-hydroxyhexyloxy)phenyl}benzene,
1-{3-methyl-4-(6-hydroxyhexyloxy)phenyl}-4-{4-(6-hydroxyhexyl oxy)phenyl}benzene,
1,4-bis{4-(8-hydroxyoctyloxy)phenyl}benzene and
1-{3-methyl-4-(8-hydroxyoctyloxy)phenyl}-4-{4-(8-hydroxyoctyl oxy)phenyl}benzene.

The dihydroxy compound (1) can be produced according to known methods described in Japanese Unexamined Patent Publication (Kokai) No. 1-168632, Japanese Unexamined Patent Publication (Kokai) No. 1-168634, US Patent specification No. 3,461,098, Japanese Unexamined Patent Publication (Kokai) No. 2-212449, Japanese Unexamined Patent Publication (Kokai) No. 2002-234856, Japanese Unexamined Patent Publication (Kokai) No. 2002-308809, Japanese Unexamined Patent Publication (Kokai) No. 2002-363117, Japanese Unexamined Patent Publication (Kokai) No. 2003-12585 and the like.

In the formula of a compound represented by the formula (2) : 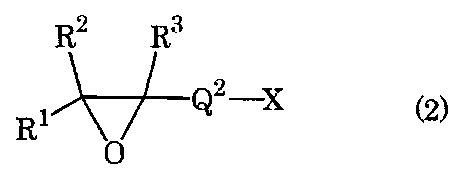 (hereinafter abbreviated to a compound (2)), R¹, R² and R³ are the same or different and each denotes hydrogen atom or an alkyl group having 1 to 8 carbon atoms, and Q² denotes a straight-chain alkylene group having 1 to 8 carbon atoms, in which at least one methylene group composing the straight-chain alkylene group is optionally substituted with an alkyl group having 1 to 8 carbon atoms.

Examples of the alkyl group having 1 to 8 carbon atoms and the straight-chain alkylene group having 1 to 8 carbon atoms include the same groups as those described above.

X denotes halogen atom and examples of the halogen atom include a chlorine atom and a bromine atom, and a chlorine atom is preferred.

Examples of the compound (2) include epichlorohydrin, epibromohydrin, 2-(chloroethyl)oxirane and
2- (bromoethyl) oxirane. If necessary, two or more kinds of them are optionally used.

The used amount of the compound (2) is usually at least 2 moles per 1 mole of the dihydroxy compound (1). The upper limit is not particularly limited and an excessive amount of the compound may be used as a solvent. In practice, the amount of the compound is 50 parts by weight or less, and preferably 20 parts by weight or less per 1 part by weight of the dihydroxy compound (1).

Examples of the amine compound include ammonia; primary amines such as methylamine, ethylamine, n-propylamine, isopropylamine, n-butylamine and isobutylamine; secondary amines such as dimethylamine, diethylamine, di-n-propylamine, diisopropylamine, di-n-butylamine, diisobutylamine, pyrrolidine, piperidine and morpholine; tertiary amines such as trimethylamine, triethylamine, tri-n-propylamine, tri-n-butylamine, N,N-dimethylaniline, N-methyl pyrrolidine, N-methylpiperidine and N-methylmorpholine; and pyridine compounds such as pyridine. Among these amine compounds, tertiary amines are preferred and triethylamine is more preferred.

Examples of the ammonium salt include a quaternary ammonium halide and a quaternary ammonium hydroxide, and a quaternary ammonium halide is preferred. Examples of the quaternary ammonium halide include quaternary ammonium chlorides such as tetraethylammonium chloride, tetra-n-butylammonium chloride and benzyltriethylammonium chloride; and quaternary ammonium bromides such as tetraethylammonium bromide and tetra-n-butylammonium bromide. Among these quaternary ammonium halides, tetraalkylammonium chloride and tetraalkylammonium bromide are preferred.

In the present invention, amine compounds may be used and ammonium salt may be used, and both amine compounds and ammonium salts may be used. Ammonium salts are preferably used. Alternatively, two or more kinds of amine compounds may be used and two or more kinds of ammonium salts may be used.

Commercially available amine compounds and ammonium salts are usually used.

The used amount thereof is usually from 0.005 to 20 moles, preferably from 0.01 to 10 moles, and more preferably from 0.05 to 5 moles per 1 mole of the dihydroxy compound (1).

The reaction of the dihydroxy compound (1) and the compound (2) may be performed in the absence or presence of a solvent. Examples of the solvent include aliphatic hydrocarbon solvents such as n-pentane, n-hexane, n-heptane, isooctane, n-octane, n-decane, cyclopentane and cyclohexane; aromatic hydrocarbon solvents such as benzene, toluene, ethylbenzene, xylene, cumene, cymene and chlorobenzene; alcohol solvents such as methanol, ethanol, n-propanol, isopropanol, n-butanol, isobutanol, n-hexanol and 2-ethylhexanol; glycol solvents such as ethylene glycol, trimethylene glycol, diethylene glycol and triethylene glycol; ketone solvents such as acetone, methyl ethyl ketone, diethyl ketone and methyl isobutyl ketone; nitrile solvents such as acetonitrile and propionitrile; ether solvents such as dimethyl ether, diethyl ether, tert-butyl methyl ether, 1,4-dioxane and tetrahydrofuran; and amide solvents such as N,N-dimethylformamide, N,N-dimethylacetamide and N-methyl-2-pyrrolidone. As described above, the compound (2) may be used as the solvent.

The used amount of the solvent is usually from 0.1 to 50 parts by weight, and preferably from 1 to 20 parts by weight, based on 1 part by weight of the dihydroxy compound (1).

The reaction of the dihydroxy compound (1) and the compound (2) is usually performed by mixing a dihydroxy compound (1), a compound (2) and at least one selected from the group consisting of an amine compound and an ammonium salt. The order of mixing them is not particularly limited. It is preferred that a dihydroxy compound (1) and a compound (2) are mixed and then at least one selected from the group consisting of an amine compound and an ammonium salt is added to the obtained mixture.

The reaction may be performed under normal pressure conditions, or may be performed under reduced pressure conditions. The reaction temperature is usually from 10 to 100°C, and preferably from 40 to 60°C. The progress of the reaction can be checked by a conventional analytical means such as liquid chromatography.

According to the present invention, a dihydroxy compound (1) and a compound (2) are reacted in the presence of at least one selected from the group consisting of an amine compound and an ammonium salt, and then the obtained reaction mixture and an inorganic base are further reacted by mixing.

The inorganic base is added to the reaction mixture after the dihydroxy compound (1) and the compound (2) were reacted and the dihydroxy compound (1) disappeared and 50% or more of a compound obtained by reacting one molecule of the obtained dihydroxy compound (1) and one molecule of the compound (2) disappeared. More preferably, the inorganic base is added to the reaction mixture after the dihydroxy compound (1) disappeared and 80% or more of a compound obtained by reacting one molecule of the obtained dihydroxy compound (1) and one molecule of the compound (2) disappeared. Particularly preferably, the inorganic base is added to the reaction mixture after the dihydroxy compound (1) disappeared and 90% or more of a compound obtained by reacting one molecule of the obtained dihydroxy compound (1) and one molecule of the compound (2) disappeared. The content of the dihydroxy compound (1) in the reaction mixture and that of the compound obtained by reacting one molecule of the dihydroxy compound (1) and one molecule of the compound (2) in the reaction mixture can be checked by a conventional analytical means such as liquid chromatography.

Examples of the inorganic base include alkali metal hydroxides such as lithium hydroxide, sodium hydroxide and potassium hydroxide; and alkali metal carbonates such as sodium carbonate and potassium carbonate. Among these inorganic bases, alkali metal hydroxides are preferred and sodium hydroxide is more preferred. The inorganic base maybe used as it is or as an aqueous solution.

The used amount of the inorganic base is usually from 0.2 to 20 moles, preferably from 1 to 15 moles, and more preferably from 1.5 to 10 moles per 1 mole of the dihydroxy compound (1) used in the reaction of the dihydroxy compound (1) and the compound (2) .

The temperature at which the inorganic base and the reaction mixture are mixed is usually from 0 to 100°C, and preferably from 10 to 50°C.

When the inorganic base is used as an aqueous solution, the reaction is preferably performed while removing water out of the reaction system, and the reaction is preferably performed at the temperature and pressure at which water is removed by azeotroping.

After completion of the reaction, an epoxy compound represented by the formula (3): 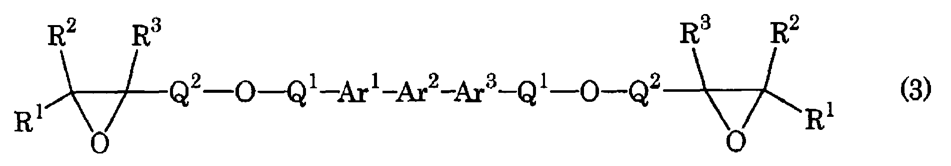 wherein Ar¹, Ar², Ar³, R¹, R², R³, Q¹ and Q² are as defined above (hereinafter abbreviated to an epoxy compound (3)) can be isolated, for example, by concentrating the obtained reaction mixture. The isolated epoxy compound (3) may be further purified by a conventional purification means such as recrystallization. Since the reaction mixture contains insolubles such as an inorganic salt, the epoxy compound (3) may be isolated by filtering the reaction mixture to remove insolubles and concentrating the reaction mixture, or the epoxy compound (3) may be isolated by adding water and a water-insoluble organic solvent if necessary to the reaction mixture thereby performing a liquid separation treatment, and concentrating the obtained organic layer.

Examples of the epoxy compound (3) thus obtained include
1,4-bis{4-(oxiranylmethoxy)phenyl}cyclohexane,
1-{2-methyl-4-(oxiranylmethoxy)phenyl}-4-{4-(oxiranylmethoxy) phenyl}cyclohexane,
1-{3-methyl-4-(oxiranylmethoxy)phenyl}-4-{4-(oxiranylmethoxy) phenyl}cyclohexane,
1-{3-ethyl-4-(oxiranylmethoxy)phenyl}-4-{4-(oxiranylmethoxy)p henyl}cyclohexane,
1-{3-n-propyl-4-(oxiranylmethoxy)phenyl}-4-{4-(oxiranylmethox y)phenyl}cyclohexane,
1-{3-isopropyl-4-(oxiranylmethoxy)phenyl}-4-{4-(oxiranylmetho xy)phenyl}cyclohexane,
1-{3-n-butyl-4-(oxiranylmethoxy)phenyl}-4-{4-(oxiranylmethoxy )phenyl}cyclohexane,
1-{3-sec-butyl-4-(oxiranylmethoxy)phenyl}-4-{4-(oxiranylmetho xy)phenyl}cyclohexane,
1-{3-tert-butyl-4-(oxiranylmethoxy)phenyl}-4-{4-(oxiranylmeth oxy)phenyl}cyclohexane,
1-{3-n-pentyl-4-(oxiranylmethoxy)phenyl}-4-{4-(oxiranylmethox y)phenyl}cyclohexane,
1-{3-tert-pentyl-4-(oxiranylmethoxy)phenyl}-4-{4-(oxiranylmet hoxy)phenyl}cyclohexane,
1-{3-n-hexyl-4-(oxiranylmethoxy)phenyl}-4-{4-(oxiranylmethoxy )phenyl}cyclohexane,
1-{3-(1-methylpentyl)-4-(oxiranylmethoxy)phenyl}-4-{4-(oxiran ylmethoxy)phenyl}cyclohexane,
1-{3-(1-ethylbutyl)-4-(oxiranylmethoxy)phenyl}-4-{4-(oxiranyl methoxy)phenyl}cyclohexane,
1-{3-cyclohexyl-4-(oxiranylmethoxy)phenyl}-4-{4-(oxiranylmeth oxy)phenyl}cyclohexane,
1-{3-n-heptyl-(oxiranylmethoxy)phenyl}-4-{4-(oxiranylmethoxy) phenyl}cyclohexane,
1-{3-n-octyl-(oxiranylmethoxy)phenyl}-4-{4-(oxiranylmethoxy)p henyl}cyclohexane,
1-{3-(2-ethylhexyl)-(oxiranylmethoxy)phenyl}-4-{4-(oxiranylme thoxy)phenyl}cyclohexane,
1-{3-tert-octyl-(oxiranylmethoxy)phenyl}-4-{4-(oxiranylmethox y)phenyl}cyclohexane,
1-{3-cyclooctyl-(oxiranylmethoxy)phenyl}-4-{4-(oxiranylmethox y)phenyl}cyclohexane,
1-{3-methyl-4-(2-methyl-oxiranylmethoxy)phenyl}-4-{4-(2-methy 1-oxiranylmethoxy)phenyl}cyclohexane,
1-{3-methyl-4-(3-methyl-oxiranylmethoxy)phenyl}-4-{4-(3-methy 1-oxiranylmethoxy)phenyl}cyclohexane,
1,4-bis{4-(oxiranylmethoxy)phenyl}-1-cyclohexene,
1-{2-methyl-4-(oxiranylmethoxy)phenyl}-4-{4-(oxiranylmethoxy) phenyl}-1-cyclohexene,
1-{3-methyl-4-(oxiranylmethoxy)phenyl}-4-{4-(oxiranylmethoxy) phenyl}-1-cyclohexene,
1-{3-ethyl-4-(oxiranylmethoxy)phenyl}-4-{4-(oxiranylmethoxy)p henyl}-1-cyclohexene,
1-{3-n-propyl-4-(oxiranylmethoxy)phenyl}-4-{4-(oxiranylmethox y)phenyl}-1-cyclohexene,
1-{3-isopropyl-4-(oxiranylmethoxy)phenyl}-4-{4-(oxiranylmetho xy)phenyl}-1-cyclohexene,
1-{3-n-butyl-4-(oxiranylmethoxy)phenyl}-4-{4-(oxiranylmethoxy )phenyl}-1-cyclohexene,
1-{3-sec-butyl-4-(oxiranylmethoxy)phenyl}-4-{4-(oxiranylmetho xy)phenyl}-1-cyclohexene,
1-{3-tert-butyl-4-(oxiranylmethoxy)phenyl}-4-{4-(oxiranylmeth oxy)phenyl}-1-cyclohexene,
1-{3-n-pentyl-4-(oxiranylmethoxy)phenyl}-4-{4-(oxiranylmethox y)phenyl}-1-cyclohexene,
1-{3-tert-pentyl-4-(oxiranylmethoxy)phenyl}-4-{4-(oxiranylmet hoxy)phenyl}-1-cyclohexene,
1-{3-n-hexyl-4-(oxiranylmethoxy)phenyl}-4-{4-(oxiranylmethoxy )phenyl}-1-cyclohexene,
1-{3-(1-methylpentyl)-4-(oxiranylmethoxy)phenyl}-4-{4-(oxiran ylmethoxy)phenyl}-1-cyclohexene,
1-{3-(1-ethylbutyl)-4-(oxiranylmethoxy)phenyl}-4-{4-(oxiranyl methoxy)phenyl}-1-cyclohexene,
1-{3-cyclohexyl-4-(oxiranylmethoxy)phenyl}-4-{4-(oxiranylmeth oxy)phenyl}-1-cyclohexene,
1-{3-n-heptyl-(oxiranylmethoxy)phenyl}-4-{9-(oxiranylmethoxy) phenyl}-1-cyclohexene,
1-{3-n-octyl-(oxiranylmethoxy)phenyl}-4-{4-(oxiranylmethoxy)p henyl}-1-cyclohexene,
1-{3-(2-ethylhexyl)-(oxiranylmethoxy)phenyl}-4-{4-(oxiranylme thoxy)phenyl}-1-cyclohexene,
1-{3-tert-octyl-(oxiranylmethoxy)phenyl}-4-{4-(oxiranylmethox y)phenyl}-1-cyclohexene,
1-{3-cyclooctyl-(oxiranylmethoxy)phenyl}-4-{4-(oxiranylmethox y)phenyl}-1-cyclohexene,
1-{3-methyl-4-(2-methyl-oxiranylmethoxy)phenyl}-4-{4-(2-methy 1-oxiranylmethoxy)phenyl}-1-cyclohexene,
1-{3-methyl-4-(3-methyl-oxiranylmethoxy)phenyl}-4-{4-(3-methy 1-oxiranylmethoxy)phenyl}-1-cyclohexene,
1,4-bis{4-(oxiranylmethoxy)phenyl}-2-cyclohexene,
1-{2-methyl-4-(oxiranylmethoxy)phenyl}-4-{4-(oxiranylmethoxy) phenyl}-2-cyclohexene,
1-{3-methyl-4-(oxiranylmethoxy)phenyl}-4-{4-(oxiranylmethoxy) phenyl}-2-cyclohexene,
1-{2-methyl-4-(oxiranylmethoxy)phenyl}-4-{4-(oxiranylmethoxy) phenyl}-2-cyclohexene,
1-{3-ethyl-4-(oxiranylmethoxy)phenyl}-4-{4-(oxiranylmethoxy)p henyl}-2-cyclohexene,
1-{3-n-propyl-4-(oxiranylmethoxy)phenyl}-4-{4-(oxiranylmethox y)phenyl}-2-cyclohexene,
1-{3-isopropyl-4-(oxiranylmethoxy)phenyl}-4-{4-(oxiranylmetho xy)phenyl}-2-cyclohexene,
1-{3-n-butyl-4-(oxiranylmethoxy)phenyl}-4-{4-(oxiranylmethoxy )phenyl}-2-cyclohexene,
1-{3-sec-butyl-4-(oxiranylmethoxy)phenyl}-4-{4-(oxiranylmetho xy)phenyl}-2-cyclohexene,
1-{3-tert-butyl-4-(oxiranylmethoxy)phenyl}-4-{4-(oxiranylmeth oxy)phenyl}-2-cyclohexene,
1-{3-n-pentyl-4-(oxiranylmethoxy)phenyl}-4-{4-(oxiranylmethox y)phenyl}-2-cyclohexene,
1-{3-tert-pentyl-4-(oxiranylmethoxy)phenyl}-4-{4-(oxiranylmet hoxy)phenyl}-2-cyclohexene,
1-{3-n-hexyl-4-(oxiranylmethoxy)phenyl}-4-{4-(oxiranylmethoxy )phenyl}-2-cyclohexene,
1-{3-(1-methylpentyl)-4-(oxiranylmethoxy)phenyl}-4-{4-(oxiran ylmethoxy)phenyl}-2-cyclohexene,
1-{3-(1-ethylbutyl)-4-(oxiranylmethoxy)phenyl}-4-{4-(oxiranyl methoxy)phenyl}-2-cyclohexene,
1-{3-cyclohexyl-4-(oxiranylmethoxy)phenyl}-4-{4-(oxiranylmeth oxy)phenyl}-2-cyclohexene,
1-{3-n-heptyl-(oxiranylmethoxy)phenyl}-4-{4-(oxiranylmethoxy) phenyl}-2-cyclohexene,
1-{3-n-octyl-(oxiranylmethoxy)phenyl}-4-{4-(oxiranylmethoxy)p henyl}-2-cyclohexene,
1-{3-(2-ethylhexyl)-(oxiranylmethoxy)phenyl}-4-{4-(oxiranylme thoxy)phenyl}-2-cyclohexene,
1-{3-tert-octyl-(oxiranylmethoxy)phenyl}-4-{4-(oxiranylmethox y)phenyl}-2-cyclohexene,
1-{3-cyclooctyl-(oxiranylmethoxy)phenyl}-4-{4-(oxiranylmethox y)phenyl}-2-cyclohexene,
1-{3-methyl-4-(2-methyl-oxiranylmethoxy)phenyl}-4-{4-(2-methy 1-oxiranylmethoxy)phenyl}-2-cyclohexene,
1-{3-methyl-4-(3-methyl-oxiranylmethoxy)phenyl}-4-{4-(3-methy 1-oxiranylmethoxy)phenyl}-2-cyclohexene,
1,4-bis{4-(oxiranylmethoxy)phenyl}-2,5-cyclohexadiene,
2-{2-methyl-4-(oxiranylmethoxy)phenyl}-4-{4-(oxiranylmethoxy) phenyl}-2,5-cyclohexadiene,
1-{3-methyl-4-(oxiranylmethoxy)phenyl}-4-{4-(oxiranylmethoxy) phenyl}-2,5-cyclohexadiene,
1-{3-ethyl-4-(oxiranylmethoxy)phenyl}-4-{4-(oxiranylmethoxy)p henyl}-2,5-cyclohexadiene,
1-{3-n-propyl-4-(oxiranylmethoxy)phenyl}-4-{4-(oxiranylmethox y)phenyl}-2,5-cyclohexadiene,
1-{3-isopropyl-4-(oxiranylmethoxy)phenyl}-4-{4-(oxiranylmetho xy)phenyl}-2,5-cyclohexadiene,
1-{3-n-butyl-4-(oxiranylmethoxy)phenyl}-4-{4-(oxiranylmethoxy )phenyl}-2,5-cyclohexadiene,
1-{3-sec-butyl-4-(oxiranylmethoxy)phenyl}-4-{4-(oxiranylmetho xy)phenyl}-2,5-cyclohexadiene,
1-{3-tert-butyl-4-(oxiranylmethoxy)phenyl}-4-{4-(oxiranylmeth oxy)phenyl}-2,5-cyclohexadiene,
1-{3-n-pentyl-4-(oxiranylmethoxy)phenyl}-4-{4-(oxiranylmethox y)phenyl}-2,5-cyclohexadiene,
1-{3-tert-pentyl-4-(oxiranylmethoxy)phenyl}-4-{4-(oxiranylmet hoxy)phenyl}-2,5-cyclohexadiene,
1-{3-n-hexyl-4-(oxiranylmethoxy)phenyl}-4-{4-(oxiranylmethoxy )phenyl}-2,5-cyclohexadiene,
1-{3-(1-methylpentyl)-4-(oxiranylmethoxy)phenyl}-4-{4-(oxiran ylmethoxy)phenyl}-2,5-cyclohexadiene,
1-{3-(1-ethylbutyl)-4-(oxiranylmethoxy)phenyl}-4-{4-(oxiranyl methoxy)phenyl}-2,5-cyclohexadiene,
1-{3-cyclohexyl-4-(oxiranylmethoxy)phenyl}-4-{4-(oxiranylmeth oxy)phenyl}-2,5-cyclohexadiene,
1-{3-n-heptyl-(oxiranylmethoxy)phenyl}-4-{4-(oxiranylmethoxy) phenyl}-2,5-cyclohexadiene,
1-{3-n-octyl-(oxiranylmethoxy)phenyl}-4-{4-(oxiranylmethoxy)p henyl}-2,5-cyclohexadiene,
1-{3-(2-ethylhexyl)-(oxiranylmethoxy)phenyl}-4-{4-(oxiranylme thoxy)phenyl}-2,5-cyclohexadiene,
1-{3-tert-octyl-(oxiranylmethoxy)phenyl}-4-{4-(oxiranylmethox y)phenyl}-2,5-cyclohexadiene,
1-{3-cyclooctyl-(oxiranylmethoxy)phenyl}-4-{4-(oxiranylmethox y)phenyl}-2,5-cyclohexadiene,
1-{3-methyl-4-(2-methyl-oxiranylmethoxy)phenyl}-4-{4-(2-methy 1-oxiranylmethoxy)phenyl}-2,5-cyclohexadiene,
1-{3-methyl-4-(3-methyl-oxiranylmethoxy)phenyl}-4-{4-(3-methy 1-oxiranylmethoxy)phenyl}-2,5-cyclohexadiene,
1,4-bis{4-(oxiranylmethoxy)phenyl}-1,5-cyclohexadiene,
1-{2-methyl-4-(oxiranylmethoxy)phenyl}-4-{4-(oxiranylmethoxy) phenyl}-1,5-cyclohexadiene,
1-{3-methyl-4-(oxiranylmethoxy)phenyl}-4-{4-(oxiranylmethoxy) phenyl}-1,5-cyclohexadiene,
1-{3-ethyl-4-(oxiranylmethoxy)phenyl}-4-{4-(oxiranylmethoxy)p henyl}-1,5-cyclohexadiene,
1-{3-n-propyl-4-(oxiranylmethoxy)phenyl}-4-{4-(oxiranylmethox y)phenyl}-1,5-cyclohexadiene,
1-{3-isopropyl-4-(oxiranylmethoxy)phenyl}-4-{4-(oxiranylmetho xy)phenyl}-1,5-cyclohexadiene,
1-{3-n-butyl-4-(oxiranylmethoxy)phenyl}-4-{4-(oxiranylmethoxy )phenyl}-1,5-cyclohexadiene,
1-{3-sec-butyl-4-(oxiranylmethoxy)phenyl}-4-{4-(oxiranylmetho xy)phenyl}-1,5-cyclohexadiene,
1-{3-tert-butyl-4-(oxiranylmethoxy)phenyl}-4-{4-(oxiranylmeth oxy)phenyl}-1,5-cyclohexadiene,
1-{3-n-pentyl-4-(oxiranylmethoxy)phenyl}-4-{4-(oxiranylmethox y)phenyl}-1,5-cyclohexadiene,
1-{3-tert-pentyl-4-(oxiranylmethoxy)phenyl}-4-{4-(oxiranylmet hoxy)phenyl}-1,5-cyclohexadiene,
1-{3-n-hexyl-4-(oxiranylmethoxy)phenyl}-4-{4-(oxiranylmethoxy )phenyl}-1,5-cyclohexadiene,
1-{3-(1-methylpentyl)-4-(oxiranylmethoxy)phenyl}-4-{4-(oxiran ylmethoxy)phenyl}-1,5-cyclohexadiene,
1-{3-(1-ethylbutyl)-4-(oxiranylmethoxy)phenyl}-4-{4-(oxiranyl methoxy)phenyl}-1,5-cyclohexadiene,
1-{3-cyclohexyl-4-(oxiranylmethoxy)phenyl}-4-{4-(oxiranylmeth oxy)phenyl}-1,5-cyclohexadiene,
1-{3-n-heptyl-(oxiranylmethoxy)phenyl}-4-{4-(oxiranylmethoxy) phenyl}-1,5-cyclohexadiene,
1-{3-n-octyl-(oxiranylmethoxy)phenyl}-4-{4-(oxiranylmethoxy)p henyl}-1,5-cyclohexadiene,
1-{3-(2-ethylhexyl)-(oxiranylmethoxy)phenyl}-4-{4-(oxiranylme thoxy)phenyl}-1,5-cyclohexadiene,
1-{3-tert-octyl-(oxiranylmethoxy)phenyl}-4-{4-(oxiranylmethox y)phenyl}-1,5-cyclohexadiene,
1-{3-cyclooctyl-(oxiranylmethoxy)phenyl}-4-{4-(oxiranylmethox y)phenyl}-1,5-cyclohexadiene,
1-{3-methyl-4-(2-methyl-oxiranylmethoxy)phenyl}-4-{4-(2-methy 1-oxiranylmethoxy)phenyl}-1,5-cyclohexadiene,
1-{3-methyl-4-(3-methyl-oxiranylmethoxy)phenyl}-4-{4-(3-methy 1-oxiranylmethoxy)phenyl}-1,5-cyclohexadiene,
1,4-bis{4-(oxiranylmethoxy)phenyl}-1,4-cyclohexadiene,
1-{2-methyl-4-(oxiranylmethoxy)phenyl}-4-{4-(oxiranylmethoxy) phenyl}-1,4-cyclohexadiene,
1-{3-methyl-4-(oxiranylmethoxy)phenyl}-4-{4-(oxiranylmethoxy) phenyl}-1,4-cyclohexadiene,
1-{3-ethyl-4-(oxiranylmethoxy)phenyl}-4-{4-(oxiranylmethoxy)p henyl}-1,4-cyclohexadiene,
1-{3-n-propyl-4-(oxiranylmethoxy)phenyl}-4-{4-(oxiranylmethox y)phenyl}-1,4-cyclohexadiene,
1-{3-isopropyl-4-(oxiranylmethoxy)phenyl}-4-{4-(oxiranylmetho xy)phenyl}-1,4-cyclohexadiene,
1-{3-n-butyl-4-(oxiranylmethoxy)phenyl}-4-{4-(oxiranylmethoxy )phenyl}-1,4-cyclohexadiene,
1-{3-sec-butyl-4-(oxiranylmethoxy)phenyl}-4-{4-(oxiranylmetho xy)phenyl}-1,4-cyclohexadiene,
1-{3-tert-butyl-4-(oxiranylmethoxy)phenyl}-4-{4-(oxiranylmeth oxy)phenyl}-1,4-cyclohexadiene,
1-{3-n-pentyl-4-(oxiranylmethoxy)phenyl}-4-{4-(oxiranylmethox y)phenyl}-1,4-cyclohexadiene,
1-{3-tert-pentyl-4-(oxiranylmethoxy)phenyl}-4-{4-(oxiranylmet hoxy)phenyl}-1,4-cyclohexadiene,
1-{3-n-hexyl-4-(oxiranylmethoxy)phenyl}-4-{4-(oxiranylmethoxy )phenyl}-1,4-cyclohexadiene,
1-{3-(1-methylpentyl)-4-(oxiranylmethoxy)phenyl}-4-{4-(oxiran ylmethoxy)phenyl}-1,4-cyclohexadiene,
1-{3-(1-ethylbutyl)-4-(oxiranylmethoxy)phenyl}-4-{4-(oxiranyl methoxy)phenyl}-1,4-cyclohexadiene,
1-{3-cyclohexyl-4-(oxiranylmethoxy)phenyl}-4-{4-(oxiranylmeth oxy)phenyl}-1,4-cyclohexadiene,
1-{3-n-heptyl-(oxiranylmethoxy)phenyl}-4-{4-(oxiranylmethoxy) phenyl}-1,4-cyclohexadiene,
1-{3-n-octyl-(oxiranylmethoxy)phenyl}-4-{4-(oxiranylmethoxy)p henyl}-1,4-cyclohexadiene,
1-{3-(2-ethylhexyl)-(oxiranylmethoxy)phenyl}-4-{4-(oxiranylme thoxy)phenyl}-1,4-cyclohexadiene,
1-{3-tert-octyl-(oxiranylmethoxy)phenyl}-4-{4-(oxiranylmethox y)phenyl}-1,4-cyclohexadiene,
1-{3-cyclooctyl-(oxiranylmethoxy)phenyl}-4-{4-(oxiranylmethox y)phenyl}-1,4-cyclohexadiene,
1-{3-methyl-4-(2-methyl-oxiranylmethoxy)phenyl}-4-{4-(2-methy 1-oxiranylmethoxy)phenyl}-1,4-cyclohexadiene,
1-{3-methyl-4-(3-methyl-oxiranylmethoxy)phenyl}-4-{4-(3-methy 1-oxiranylmethoxy)phenyl}-1,4-cyclohexadiene,
1,4-bis{4-(oxiranylmethoxy)phenyl}-1,3-cyclohexadiene,
1-{2-methyl-4-(oxiranylmethoxy)phenyl}-4-{4-(oxiranylmethoxy) phenyl}-1,3-cyclohexadiene,
1-{3-methyl-4-(oxiranylmethoxy)phenyl}-4-{4-(oxiranylmethoxy) phenyl}-1,3-cyclohexadiene,
1-{3-ethyl-4-(oxiranylmethoxy)phenyl}-9-{4-(oxiranylmethoxy)p henyl}-1,3-cyclohexadiene,
1-{3-n-propyl-4-(oxiranylmethoxy)phenyl}-4-{4-(oxiranylmethox y)phenyl}-1,3-cyclohexadiene,
1-{3-isopropyl-4-(oxiranylmethoxy)phenyl}-4-{4-(oxiranylmetho xy)phenyl}-1,3-cyclohexadiene,
1-{3-n-butyl-4-(oxiranylmethoxy)phenyl}-4-{4-(oxiranylmethoxy )phenyl}-1,3-cyclohexadiene,
1-{3-sec-butyl-4-(oxiranylmethoxy)phenyl}-4-{4-(oxiranylmetho xy)phenyl}-1,3-cyclohexadiene,
1-{3-tert-butyl-4-(oxiranylmethoxy)phenyl}-4-{9-(oxiranylmeth oxy)phenyl}-1,3-cyclohexadiene,
1-{3-n-pentyl-4-(oxiranylmethoxy)phenyl}-4-{4-(oxiranylmethox y)phenyl}-1,3-cyclohexadiene,
1-{3-tert-pentyl-4-(oxiranylmethoxy)phenyl}-4-{4-(oxiranylmet hoxy)phenyl}-1,3-cyclohexadiene,
1-{3-n-hexyl-4-(oxiranylmethoxy)phenyl}-4-{4-(oxiranylmethoxy )phenyl}-1,3-cyclohexadiene,
1-{3-(1-methylpentyl)-4-(oxiranylmethoxy)phenyl}-4-{4-(oxiran ylmethoxy)phenyl}-1,3-cyclohexadiene,
1-{3-(1-ethylbutyl)-4-(oxiranylmethoxy)phenyl}-4-{4-(oxiranyl methoxy)phenyl}-1,3-cyclohexadiene,
1-{3-cyclohexyl-4-(oxiranylmethoxy)phenyl}-4-{4-(oxiranylmeth oxy)phenyl}-1,3-cyclohexadiene,
1-{3-n-heptyl-(oxiranylmethoxy)phenyl}-4-{4-(oxiranylmethoxy) phenyl}-1,3-cyclohexadiene,
1-{3-n-octyl-(oxiranylmethoxy)phenyl}-4-{4-(oxiranylmethoxy)p henyl}-1,3-cyclohexadiene,
1-{3-(2-ethylhexyl)-(oxiranylmethoxy)phenyl}-4-{4-(oxiranylme thoxy)phenyl}-1,3-cyclohexadiene,
1-{3-tert-octyl-(oxiranylmethoxy)phenyl}-4-{4-(oxiranylmethox y)phenyl}-1,3-cyclohexadiene,
1-{3-cyclooctyl-(oxiranylmethoxy)phenyl}-4-{4-(oxiranylmethox y)phenyl}-1,3-cyclohexadiene,
1-{3-methyl-4-(2-methyl-oxiranylmethoxy)phenyl}-4-{4-(2-methy 1-oxiranylmethoxy)phenyl}-1,3-cyclohexadiene,
1-{3-methyl-4-(3-methyl-oxiranylmethoxy)phenyl}-4-{4-(3-methy 1-oxiranylmethoxy)phenyl}-1,3-cyclohexadiene,
1,4-bis{4-(oxiranylmethoxy)phenyl}benzene,
1-{2-methyl-4-(oxiranylmethoxy)phenyl}-4-{4-(oxiranylmethoxy) phenyl}benzene,
1-{3-methyl-4-(oxiranylmethoxy)phenyl}-4-{4-(oxiranylmethoxy) phenyl}benzene,
1-{3-ethyl-4-(oxiranylmethoxy)phenyl}-4-{4-(oxiranylmethoxy)p henyl}benzene,
1-{3-n-propyl-4-(oxiranylmethoxy)phenyl}-4-{4-(oxiranylmethox y)phenyl}benzene,
1-{3-isopropyl-4-(oxiranylmethoxy)phenyl}-4-{4-(oxiranylmetho xy)phenyl}benzene,
1-{3-n-butyl-4-(oxiranylmethoxy)phenyl}-4-{4-(oxiranylmethoxy )phenyl}benzene,
1-{3-sec-butyl-4-(oxiranylmethoxy)phenyl}-4-{4-(oxiranylmetho xy)phenyl}benzene,
1-{3-tert-butyl-4-(oxiranylmethoxy)phenyl}-4-{4-(oxiranylmeth oxy)phenyl}benzene,
1-{3-n-pentyl-4-(oxiranylmethoxy)phenyl}-4-{4-(oxiranylmethox y)phenyl}benzene,
1-{3-tert-pentyl-4-(oxiranylmethoxy)phenyl}-4-{4-(oxiranylmet hoxy)phenyl}benzene,
1-{3-n-hexyl-4-(oxiranylmethoxy)phenyl}-4-{4-(oxiranylmethoxy )phenyl}benzene,
1-{3-(1-methylpentyl)-4-(oxiranylmethoxy)phenyl}-4-{4-(oxiran ylmethoxy)phenyl}benzene,
1-{3-(1-ethylbutyl)-4-(oxiranylmethoxy)phenyl}-4-{4-(oxiranyl methoxy)phenyl}benzene,
1-{3-cyclohexyl-4-(oxiranylmethoxy)phenyl}-4-{4-(oxiranylmeth oxy)phenyl}benzene,
1-{3-n-heptyl-(oxiranylmethoxy)phenyl}-4-{4-(oxiranylmethoxy) phenyl}benzene,
1-{3-n-octyl-(oxiranylmethoxy)phenyl}-4-{4-(oxiranylmethoxy)p henyl}benzene,
1-{3-(2-ethylhexyl)-(oxiranylmethoxy)phenyl}-4-{4-(oxiranylme thoxy)phenyl}benzene,
1-{3-tert-octyl-(oxiranylmethoxy)phenyl}-4-{4-(oxiranylmethox y)phenyl}benzene,
1-{3-cyclooctyl-(oxiranylmethoxy)phenyl}-4-{4-(oxiranylmethox y)phenyl}benzene,
1-{3-methyl-4-(2-methyl-oxiranylmethoxy)phenyl}-4-{4-(2-methy 1-oxiranylmethoxy)phenyl}benzene,
1-{3-methyl-4-(3-methyl-oxiranylmethoxy)phenyl}-4-{4-(3-methy 1-oxiranylmethoxy)phenyl}benzene,
1,4-bis{4-(oxiranylmethoxyethoxy)phenyl}cyclohexane,
1-{3-methyl-4-(oxiranylmethoxyethoxy)phenyl}-4-{4-(oxiranylme thoxyethoxy)phenyl}cyclohexane,
1,4-bis{4-(3-oxiranylpropoxy)phenyl}cyclohexane,
1-{3-methyl-4-(3-oxiranylpropoxy)phenyl}-4-{4-(3-oxiranylprop oxy)phenyl}cyclohexane,
1,4-bis{4-(4-oxiranylbutoxy)phenyl}cyclohexane,
1-{3-methyl-4-(4-oxiranylbutoxy)phenyl}-4-{4-(4-oxiranylbutox y)phenyl}cyclohexane,
1,4-bis{4-(5-oxiranylpentyloxy)phenyl}cyclohexane,
1-{3-methyl-4-(5-oxiranylpentyloxy)phenyl}-4-{4-(5-oxiranylpe ntyloxy)phenyl}cyclohexane,
1,4-bis{4-(6-oxiranylhexyloxy)phenyl}cyclohexane,
1-{3-methyl-4-(6-oxiranylhexyloxy)phenyl}-4-{4-(6-oxiranylhex yloxy)phenyl}cyclohexane,
1,4-bis{4-(8-oxiranyloctyloxy)phenyl}cyclohexane,
1-{3-methyl-4-(8-oxiranyloctyloxy)phenyl}-4-{4-(8-oxiranyloct yloxy)phenyl}cyclohexane,
1,4-bis{4-(oxiranylmethoxyethoxy)phenyl}cyclohexane,
1-{3-methyl-4-(oxiranylmethoxyethoxy)phenyl}-4-{4-(oxiranylme thoxyethoxy)phenyl}cyclohexane,
1,4-bis{4-(2-methyl-oxiranyl)methoxyethoxyphenyl}cyclohexane,
1-{3-methyl-4-(2-methyl-oxiranyl)methoxyethoxyphenyl}-4-{4-(2 -methyl-oxiranyl)methoxyethoxyphenyl}cyclohexane,
1,4-bis{4-(3-methyl-oxiranyl)methoxyethoxyphenyl}cyclohexane,
1-{3-methyl-4-(3-methyl-oxiranyl)methoxyethoxyphenyl}-4-{4-(3 -methyl-oxiranyl)methoxyethoxyphenyl}cyclohexane,
1,4-bis{4-(oxiranylethoxy)phenyl}-1-cyclohexene,
1-{3-methyl-4-(oxiranylethoxy)phenyl}-4-{4-(oxiranylethoxy)ph enyl}-1-cyclohexene,
1,4-bis{4-(3-oxiranylpropoxy)phenyl}-1-cyclohexene,
1-{3-methyl-4-(3-oxiranylpropoxy)phenyl}-4-{4-(3-oxiranylprop oxy)phenyl}-1-cyclohexene,
1,4-bis{4-(4-oxiranylbutoxy)phenyl}-1-cyclohexene,
1-{3-methyl-4-(4-oxiranylbutoxy)phenyl}-4-{4-(4-oxiranylbutox y)phenyl}-1-cyclohexene,
1,4-bis{4-(5-oxiranylpentyloxy)phenyl}-1-cyclohexene,
1-{3-methyl-4-(5-oxiranylpentyloxy)phenyl}-4-{4-(5-oxiranylpe ntyloxy)phenyl}-1-cyclohexene,
1,4-bis{4-(6-oxiranylhexyloxy)phenyl}-1-cyclohexene,
1-{3-methyl-4-(6-oxiranylhexyloxy)phenyl}-4-{4-(6-oxiranylhex yloxy)phenyl}-1-cyclohexene,
1,4-bis{4-(8-oxiranyloctyloxy)phenyl}-1-cyclohexene,
1-{3-methyl-4-(8-oxiranyloctyloxy)phenyl}-4-{4-(8-oxiranyloct yloxy)phenyl}-1-cyclohexene,
1,4-bis{4-(oxiranylmethoxyethoxy)phenyl}-1-cyclohexene,
1-{3-methyl-4-(oxiranylmethoxyethoxy)phenyl}-4-{4-(oxiranylme thoxyethoxy)phenyl}-1-cyclohexene,
1,4-bis{4-(2-methyl-oxiranyl)methoxyethoxyphenyl}-1-cyclohexe ne,
1-{3-methyl-4-(2-methyl-oxiranyl)methoxyethoxyphenyl}-4-{4-(2 -methyl-oxiranyl)methoxyethoxyphenyl}-1-cyclohexene,
1,4-bis{4-(3-methyl-oxiranyl)methoxyethoxyphenyl}-1-cyclohexe ne,
1-{3-methyl-4-(3-methyl-oxiranyl)methoxyethoxyphenyl}-4-{4-(3 -methyl-oxiranyl)methoxyethoxyphenyl}-1-cyclohexene,
1,4-bis{4-(oxiranylmethoxyethoxy)phenyl}benzene,
1-{3-methyl-4-(oxiranylmethoxyethoxy)phenyl}-4-{4-(oxiranylme thoxyethoxy)phenyl}benzene,
1,4-bis{4-(3-oxiranylpropoxy)phenyl}benzene,
1-{3-methyl-4-(3-oxiranylpropoxy)phenyl}-4-{4-(3-oxiranylprop oxy)phenyl}benzene,
1,4-bis{4-(4-oxiranylbutoxy)phenyl}benzene,
1-{3-methyl-4-(4-oxiranylbutoxy)phenyl}-4-{4-(4-oxiranylbutox y)phenyl}benzene,
1,4-bis{4-(5-oxiranylpentyloxy)phenyl}benzene,
1-{3-methyl-4-(5-oxiranylpentyloxy)phenyl}-4-{4-(5-oxiranylpe ntyloxy)phenyl}benzene,
1,4-bis{4-(6-oxiranylhexyloxy)phenyl}benzene,
1-{3-methyl-4-(6-oxiranylhexyloxy)phenyl}-4-{4-(6-oxiranylhex yloxy)phenyl}benzene,
1,4-bis{4-(8-oxiranyloctyloxy)phenyl}benzene,
1-{3-methyl-4-(8-oxiranyloctyloxy)phenyl}-4-{4-(8-oxiranyloct yloxy)phenyl}benzene,
1,4-bis{4-(oxiranylmethoxyethoxy)phenyl}benzene,
1-{3-methyl-4-(oxiranylmethoxyethoxy)phenyl}-4-{4-(oxiranylme thoxyethoxy)phenyl}benzene,
1,4-bis{4-(2-methyl-oxiranyl)methoxyethoxyphenyl}benzene,
1-{3-methyl-4-(2-methyl-oxiranyl)methoxyethoxyphenyl}-4-{4-(2 -methyl-oxiranyl)methoxyethoxyphenyl}benzene,
1,4-bis{4-(3-methyl-oxiranyl)methoxyethoxyphenyl}benzene and
1-{3-methyl-4-(3-methyl-oxiranyl)methoxyethoxyphenyl}-4-{4-(3 -methyl-oxiranyl)methoxyethoxyphenyl}benzene.

### Examples

Hereinafter, the present invention is further illustrated in detail by referring to Examples, but the present invention is not limited to Examples. Analysis was performed by high-performance liquid chromatography and purity of the obtained epoxy compound was calculated by a high-performance liquid chromatography area percentage method.

### Example 1

25.0 g of
1-(3-methyl-4-hydroxyphenyl)-4-(4-hydroxyphenyl)-1-cyclohexen e, 247.8 g of epichlorohydrin and 1.815 g of triethylamine were placed in a reaction vessel equipped with a thermometer, a stirrer and a condenser tube. Under a nitrogen atmosphere, the obtained mixture was heated at 50°C for 11 hours while stirring. The reaction mixture was analyzed by high-performance liquid chromatography. As a result,
1-(3-methyl-4-hydroxyphenyl)-4-(4-hydroxyphenyl)-1-cyclohexen e disappeared and an area percentage value of a compound obtained by reacting one molecule of
1-(3-methyl-4-hydroxyphenyl)-4-(4-hydroxyphenyl)-1-cyclohexen and one molecule of epichlorohydrin was 1.01%. The obtained reaction mixture was cooled to room temperature and 11.0 g of sodium hydroxide was added thereto, and then the mixture was further reacted at room temperature for 2 hours to obtain a reaction mixture containing
1-(3-methyl-4-oxiranylmethoxyphenyl)-4-(4-oxiranylmethoxyphen yl)-1-cyclohexene. The reaction mixture was analyzed by high-performance liquid chromatography. As a result, an area percentage value of
1-(3-methyl-4-oxiranylmethoxyphenyl)-4-(4-oxiranylmethoxyphen yl)-1-cyclohexene was 96.0%.

### Example 2

100.1 g of
1-(3-methyl-4-hydroxyphenyl)-4-(4-hydroxyphenyl)-1-cyclohexen e, 980. 6 g of epichlorohydrin and 23.8 g of tetra-n-butylammonium bromide were placed in a reaction vessel equipped with a thermometer, a stirrer and a condenser tube. Under a nitrogen atmosphere, the obtained mixture was heated at 50°C for 10 hours while stirring. The reaction mixture was analyzed by high-performance liquid chromatography. As a result, 1-(3-methyl-4-hydroxyphenyl)-4-(4-hydroxyphenyl)-1-cyclohexen e disappeared and an area percentage value of a compound obtained by reacting one molecule of
1-(3-methyl-4-hydroxyphenyl)-4-(4-hydroxyphenyl)-1-cyclohexen e and one molecule of epichlorohydrin was 0.97%. The obtained reaction mixture was cooled to room temperature and 43.8 g of sodium hydroxide was added thereto, and then the mixture was further reacted at room temperature for 2 hours to obtain a reaction mixture containing
1-(3-methyl-4-oxiranylmethoxyphenyl)-4-(4-oxiranylmethoxyphen yl)-1-cyclohexene. The reaction mixture was analyzed by high-performance liquid chromatography. As a result, an area percentage value of
1-(3-methyl-4-oxiranylmethoxyphenyl)-4-(4-oxiranylmethoxyphen yl)-1-cyclohexene was 96.6%.

To the obtained reaction mixture, 400 g of ion-exchange water was added, followed by stirring at 50°C for 30 minutes. After standing, an organic layer and an aqueous layer were separated. The obtained organic layer was concentrated under reduced pressure conditions at 50°C to obtain 195.7 g of 1-(3-methyl-4-oxiranylmethoxyphenyl)-4-(4-oxiranylmethoxyphen yl)-1-cyclohexene. Purity was 96.2%.

49.1 g of the obtained
1-(3-methyl-4-oxiranylmethoxyphenyl)-4-(4-oxiranylmethoxyphen yl) -1-cyclohexene was dissolved in 217 g of methyl isobutyl ketone at 50°C. The obtained solution was cooled to 10°C over 3.5 hours and then maintained at the same temperature for 2 hours. The obtained slurry was filtered. The obtained solid was washed with 20 g of methyl isobutyl ketone adjusted to 10°C and then dried under reduced pressure conditions at 50°C to obtain 26.6 g of 1-(3-methyl-4-oxiranylmethoxyphenyl)-4-(4-oxiranylmethoxyphen yl)-1-cyclohexene as a white solid. Purity was 97.5% and yield was 74.8%.

### Reference Example 1

1,042 g of the reaction mixture containing 1-(3-methyl-4-oxiranylmethoxyphenyl)-4-(4-oxiranylmethoxyphen yl)-1-cyclohexene obtained in the same manner as in Example 2 (an area percentage value of
1-(3-methyl-4-oxiranylmethoxyphenyl)-4-(4-oxiranylmethoxyphen yl)-1-cyclohexene: 96.2%) and 400 g of ion-exchange water were mixed and the obtained solution was stirred at 50°C for 30 minutes. After standing, an organic layer and an aqueous layer were separated. The organic layer was further washed twice with ion-exchange water. The organic layer was concentrated under reduced pressure conditions at 50°C and 423 g of methanol was added to the obtained concentrated solution, followed by cooling to 10°C over 2 hours. After maintaining at 10°C for 2 hours, the obtained slurry was filtered. The obtained solid was washed four times with 80% methanol water and then the obtained solid was washed twice with ion-exchange water. The obtained solid was dried under reduced pressure conditions at 60°C to obtain 87.2 g of
1-(3-methyl-4-oxiranylmethoxyphenyl)-4-(4-oxiranylmethoxyphen yl)-1-cyclohexene as a white solid. Purity was 96.5% and yield was 87.6%.

### Comparative Example 1

25.0 g of
1-(3-methyl-4-hydroxyphenyl)-4-(4-hydroxyphenyl)-1-cyclohexen e, 246.5 g of epichlorohydrin and 7.7 g of sodium hydroxide were placed in a reaction vessel equipped with a thermometer, a stirrer and a condenser tube. The obtained mixture was reacted by heating at 50°C for 2 hours while stirring. The reaction mixture was analyzed by high-performance liquid chromatography. As a result, an area percentage value of
1-(3-methyl-4-oxiranylmethoxyphenyl)-4-(4-oxiranylmethoxyphen yl)-1-cyclohexene was 8.8%.

To the reaction mixture, 3.3 g of sodium hydroxide was added and then the reaction was performed at 50°C for 2 hours to obtain the reaction mixture containing
1-(3-methyl-4-oxiranylmethoxyphenyl)-4-(4-oxiranylmethoxyphen yl)-1-cyclohexene. The reaction mixture was analyzed by high-performance liquid chromatography. As a result, an area percentage value of
1-(3-methyl-4-oxiranylmethoxyphenyl)-4-(4-oxiranylmethoxyphen yl)-1-cyclohexene was 87.4%.

### Comparative Example 2

25.0 g of
1-(3-methyl-4-hydroxyphenyl)-4-(4-hydroxyphenyl)-1-cyclohexen e, 247.7 g of epichlorohydrin and 24.7 g of potassium carbonate were placed in a reaction vessel equipped with a thermometer, a stirrer and a condenser tube. The obtained mixture was reacted by heating at 50°C for 10 hours while stirring. The reaction mixture was analyzed by high-performance liquid chromatography. As a result, an area percentage value of
1-(3-methyl-4-oxiranylmethoxyphenyl)-4-(4-oxiranylmethoxyphen yl)-1-cyclohexene was 0.8%.

To the reaction mixture, 24.6 g of potassium carbonate was further added and then the reaction was performed by heating at 50°C for 18 hours to obtain a the reaction mixture containing 1-(3-methyl-4-oxiranylmethoxyphenyl)-4-(4-oxiranylmethoxyphen yl)-1-cyclohexene. The reaction mixture was analyzed by high-performance liquid chromatography. As a result, an area percentage value of
1-(3-methyl-4-oxiranylmethoxyphenyl)-4-(4-oxiranylmethoxyphen yl)-1-cyclohexene was 10.1%.

To the reaction mixture, 7.4 g of sodium hydroxide was added and then the reaction was performed by heating at room temperature for 4 hours to obtain a reaction mixture containing 1-(3-methyl-4-oxiranylmethoxyphenyl)-4-(4-oxiranylmethoxyphen yl)-1-cyclohexene. The reaction mixture was analyzed by high-performance liquid chromatography. As a result, an area percentage value of
1-(3-methyl-4-oxiranylmethoxyphenyl)-4-(4-oxiranylmethoxyphen yl)-1-cyclohexene was 89.8%.

### Comparative Example 3

1 g of
1-(3-methyl-4-hydroxyphenyl)-4-(4-hydroxyphenyl)-1-cyclohexen e, 9.9 g of epichlorohydrin, 0.23 g of tetra-n-butylammonium bromide and 0.97 g of sodium hydroxide were placed in a reaction vessel equipped with a thermometer, a stirrer and a condenser tube. The obtained mixture was reacted by heating at 50°C for 2 hours while stirring. The reaction mixture was analyzed by high-performance liquid chromatography. As a result, an area percentage value of
1-(3-methyl-4-oxiranylmethoxyphenyl)-4-(4-oxiranylmethoxyphen yl)-1-cyclohexene was 89.7%.

### Comparative Example 4

0.5.g of
1-(3-methyl-4-hydroxyphenyl)-4-(4-hydroxyphenyl)-1-cyclohexen e, 4.95 g of epichlorohydrin, 0.115 g of tetra-n-butylammonium bromide and 0.21 g of sodium hydroxide were placed in a reaction vessel equipped with a thermometer, a stirrer and a condenser tube. The obtained mixture was reacted by heating at 25°C for 24 hours. The reaction mixture was analyzed by high-performance liquid chromatography. As a result, an area percentage value of 1-(3-methyl-4-oxiranylmethoxyphenyl)-4-(4-oxiranylmethoxyphen yl)-1-cyclohexene was 85.6%.

### Industrial Applicability

According to the present invention, an epoxy compound represented by the formula (3), capable of forming a cured resin which is useful as an insulating material requiring high thermal conductivity, can be produced with higher purity in a high yield. Therefore, the present invention is industrially advantageous.

## Claims

1. A process for producing an epoxy compound represented by the formula (3): 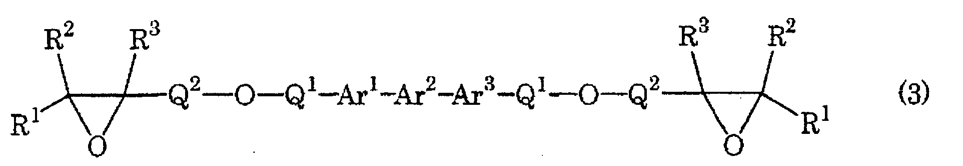 wherein Ar¹, Ar², Ar³, R¹, R², R³, Q¹ and Q² are as defined blow, which comprises reacting a dihydroxy compound represented by the formula (1):
HO-Q¹-Ar¹-Ar²-Ar³-Q¹-OH (1)
wherein,
Q¹ denotes a single bond or a straight-chain alkylene group having 1 to 9 carbon atoms, in which at least one methylene group composing the straight-chain alkylene group is optionally substituted with an alkyl group having 1 to 8 carbon atoms;
Ar¹, Ar² and Ar³ are the same or different and each denotes any one of divalent groups represented by the following formulas: 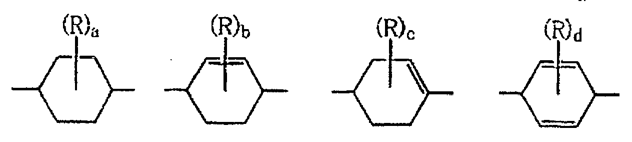 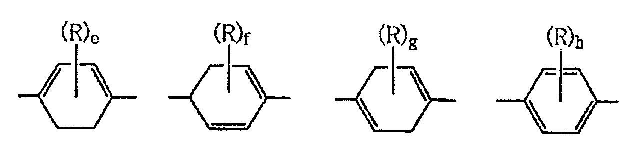 in which R denotes an alkyl group having 1 to 8 carbon atoms, a denotes an integer of 0 to 8, b, e and g denote an integer of 0 to 6, c denotes an integer of 0 to 7, d and h denote an integer of 0 to 4, f denotes an integer of 0 to 5,and when more than one R exists in said divalent group, all of R may be the same group or different groups; and a compound represented by the formula (2) : 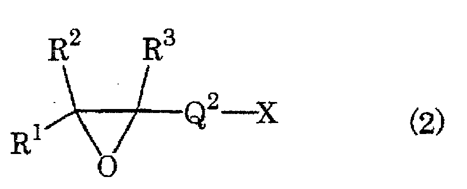 wherein R¹, R² and R³ are the same or different and each denotes a hydrogen atom or an alkyl group having 1 to 8 carbon atoms, Q² denotes a straight-chain alkylene group having 1 to 8 carbon atoms, in which at least one methylene group composing the straight-chain alkylene group is optionally substituted with an alkyl group having 1 to 8 carbon atoms, and X denotes a halogen atom, in the presence of at least one selected from the group consisting of an amine compound and an ammonium salt, and further reacting the obtained reaction mixture and an inorganic base by mixing, wherein the inorganic base is added to the reaction mixture after the dihydroxy compound represented by the formula (1) and the compound represented by the formula (2) were reacted and the dihydroxy compound represented by the formula (1) disappeared and 50% or more of a compound obtained by reacting one molecule of the dihydroxy compound represented by the formula (1) and one molecule of the compound represented by the formula (2) disappeared.

2. The process for producing an epoxy compound according to claim 1, wherein an inorganic base is added to the reaction mixture after the dihydroxy compound represented by the formula (1) disappeared and 80% or more of a compound obtained by reacting one molecule of the dihydroxy compound represented by the formula (1) and one molecule of the compound represented by the formula (2) disappeared.

3. The process for producing an epoxy compound according to claim 1, wherein an inorganic base is added to the reaction mixture after the dihydroxy compound represented by the formula (1) disappeared and 90% or more of a compound obtained by reacting one molecule of the dihydroxy compound represented by the formula (1) and one molecule of the compound represented by the formula (2) disappeared.

4. The process for producing an epoxy compound according to any one of claims 1 to 3, wherein the dihydroxy compound represented by the formula (1) and the compound represented by the formula (2) are reacted in the presence of an ammonium salt.

5. The process for producing an epoxy compound according to any one of claims 1 to 3, wherein the amine compound is a tertiary amine.

6. The process for producing an epoxy compound according to claim 5, wherein the tertiary amine is triethylamine.

7. The process for producing an epoxy compound according to any one of claims 1 to 3, wherein the ammonium salt is a quaternary ammonium salt.

8. The process for producing an epoxy compound according to claim 7, wherein the quaternary ammonium salt is a quaternary ammonium halide.

9. The process for producing an epoxy compound according to claim 8, wherein the quaternary ammonium halide is tetraalkylammonium chloride or tetraalkylammonium bromide.

10. The process for producing an epoxy compound according to any one of claims 1 to 3, wherein the inorganic base is an alkali metal hydroxide.

11. The process for producing an epoxy compound according to any one of claims 1 to 3, wherein Ar¹ and Ar³ independently denote a group represented by the following formula: 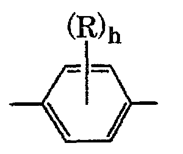 wherein R and h are as defined above.

12. The process for producing an epoxy compound according to claim 11, wherein Ar¹ and Ar³ independently denote a 1, 4-phenylene group, 3-methyl-1,4-phenylene group or 3-isopropyl-1,4-phenylene group.

13. The process for producing an epoxy compound according to any one of claims 1 to 3, wherein Ar² is a group represented by the following formula: 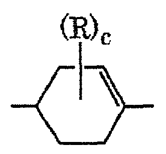 wherein R and c are as defined above.

14. The process for producing an epoxy compound according to claim 13, wherein Ar² is a 1-cyclohexene-1,4-diyl group.

15. The process for producing an epoxy compound according to any one of claims 1 to 3, wherein Ar¹ and Ar³ independently denote a group represented by the following formula: 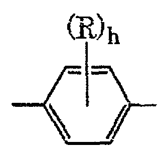 wherein R and h are as defined above, and Ar² denotes a group represented by the following formula: 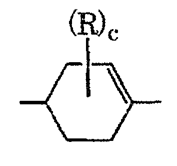 wherein R and c are as defined above.

16. The process for producing an epoxy compound according to claim 15, wherein Ar¹ and Ar³ independently denote a 1, 4-phenylene group, 3-methyl-1,4-phenylene group or 3-isopropyl-1,4-phenylene group, and Ar² denotes a 1-cyclohexene-1,4-diyl group.

## Patentansprüche

1. Ein Verfahren zur Herstellung einer Epoxyverbindung der Formel (3): 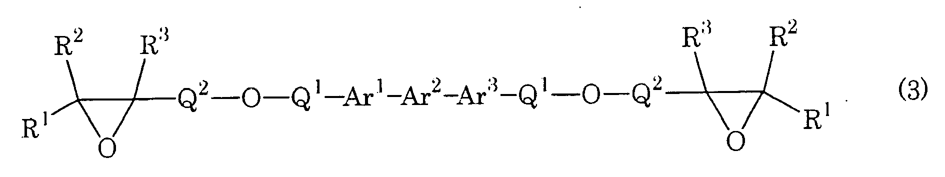 wobei Ar¹, Ar², Ar³, R¹, R², R³, Q¹ und Q² wie unten definiert sind,
welches Umsetzen einer Dihydroxyverbindung der Formel (1):
HO-Q¹-Ar¹-Ar²-Ar³-Q¹-OH (1)
wobei,
Q¹ eine Einfachbindung oder einen geradkettigen Alkylenrest mit 1 bis 9 Kohlenstoffatomen, in welchem mindestens eine den geradkettigen Alkylenrest bildende Methylengruppe gegebenenfalls mit einem 1 bis 8 Kohlenstoffatome aufweisenden Alkylrest substituiert ist, bedeutet;
Ar¹, Ar² und Ar³ gleich oder verschieden sind und jeweils einen der zweiwertigen Reste der folgenden Formeln bedeuten: 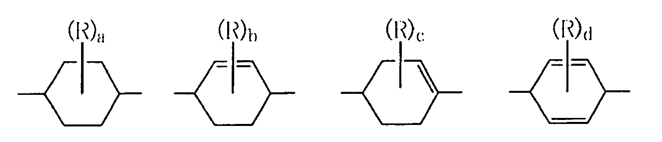 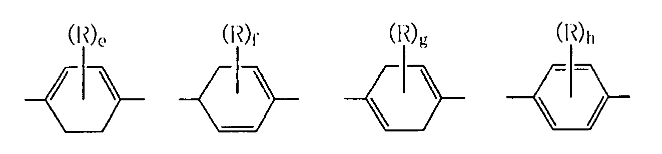 in welchen R einen Alkylrest mit 1 bis 8 Kohlenstoffatomen aufweist bedeutet, a eine ganze Zahl von 0 bis 8 bedeutet, b, e und g eine ganze Zahl von 0 bis 6 bedeuten, c eine ganze Zahl von 0 bis 7 bedeutet, d und h eine ganze Zahl von 0 bis 4 bedeuten, f eine ganze Zahl von 0 bis 5 bedeutet, und wenn mehr als ein R in dem zweiwertigen Rest vorhanden ist, alle R der gleiche oder verschiedene Reste sein können; und einer Verbindung der Formel (2): 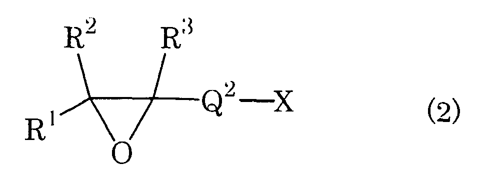 wobei R¹, R² und R³ gleich oder verschieden sind, und jeweils ein Wasserstoffatom oder einen Alklyrest mit 1 bis 8 Kohlenstoffatomen aufweist bedeuten, Q² einen geradkettigen Alkylenrest mit 1 bis 8 Kohlenstoffatomen aufweist, in welchen mindestens eine den geradkettigen Alkylrest bildende Methylengruppe gegebenenfalls mit einem 1 bis 8 Kohlenstoffatome aufweisenden Alkylrest substituiert ist, bedeutet, und X ein Halogenatom bedeutet, in der Gegenwart von mindestens einem, ausgewählt aus der Gruppe bestehend aus einer Aminverbindung und einem Ammoniumsalz, und weiteres Umsetzen des erhaltenen Reaktionsgemisches und einer anorganischen Base durch Vermischen umfasst, wobei die anorganische Base dem Reaktionsgemisch zugegeben wird, nachdem die Dihydroxyverbindung der Formel (1) und die Verbindung der Formel (2) umgesetzt wurden, und die Dihydroxyverbindung der Formel (1) verschwunden ist, und 50 % oder mehr von einer durch Umsetzen eines Moleküls der Dihydroxyverbindung dargestellt durch die Formel (1) und eines Moleküls der Verbindung der Formel (2) erhaltenen Verbindung verschwunden sind.

2. Das Verfahren zur Herstellung einer Epoxyverbindung gemäß Anspruch 1, wobei eine anorganische Base dem Reaktionsgemisch zugegeben wird, nachdem die Dihydroxyverbindung der Formel (1) verschwunden ist, und 80 % oder mehr einer durch Umsetzen eines Moleküls der Dihydroxyverbindung der Formel (1) und eines Moleküls der Verbindung der Formel (2) erhaltenen Verbindung verschwunden sind.

3. Das Verfahren zur Herstellung einer Epoxyverbindung gemäß Anspruch 1, wobei eine anorganische Base dem Reaktionsgemisch zugegeben wird, nachdem die Dihydroxyverbindung der Formel (1) verschwunden ist, und 90 % oder mehr durch Umsetzen eines Moleküls der Dihydroxyverbindung der Formel (1) und eines Moleküls der Verbindung der Formel (2) erhaltenen Verbindung verschwunden sind.

4. Das Verfahren zur Herstellung einer Epoxyverbindung gemäß einem der Ansprüche 1 bis 3, wobei die Dihydroxyverbindung der Formel (1) und die Verbindung der Formel (2) in Gegenwart eines Ammoniumsalzes umgesetzt werden.

5. Das Verfahren zur Herstellung einer Epoxyverbindung gemäß einem der Ansprüche 1 bis 3, wobei die Aminverbindung ein tertiäres Amin ist.

6. Das Verfahren zur Herstellung einer Epoxyverbindung gemäß Anspruch 5, wobei das tertiäre Amin Triethylamin ist.

7. Das Verfahren zur Herstellung einer Epoxyverbindung gemäß einem der Ansprüche 1 bis 3, wobei das Ammoniumsalz ein quartäres Ammoniumsalz ist.

8. Das Verfahren zur Herstellung einer Epoxyverbindung gemäß Anspruch 7, wobei das quartäre Ammoniumsalz ein quartäres Ammoniumhalogenid ist.

9. Das Verfahren zur Herstellung einer Epoxyverbindung gemäß Anspruch 8, wobei das quartäre Ammoniumhalogenid Tetraalkylammoniumchlorid oder Tetraalkylammoniumbromid ist.

10. Das Verfahren zur Herstellung einer Epoxyverbindung gemäß einem der Ansprüche 1 bis 3, wobei die anorganische Base ein Alkalimetallhydroxid ist.

11. Das Verfahren zur Herstellung einer Epoxyverbindung gemäß einem der Ansprüche 1 bis 3, wobei Ar¹ und Ar³ unabhängig einen Rest der folgenden Formel bedeuten: 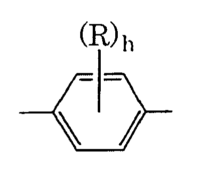 wobei R und h wie oben definiert sind.

12. Das Verfahren zur Herstellung einer Epoxyverbindung gemäß Anspruch 11, wobei Ar¹ und Ar³ unabhängig eine 1,4-Phenylengruppe, eine 3-Methyl-1,4-phenylengruppe oder eine 3-Isopropyl-1,4-phenylengruppe bedeuten.

13. Das Verfahren zur Herstellung einer Epoxyverbindung gemäß einem der Ansprüche 1 bis 3, wobei Ar² ein Rest der folgenden Formel ist: 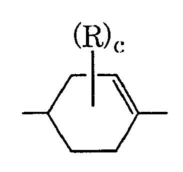 wobei R und c wie oben definiert sind.

14. Das Verfahren zur Herstellung einer Epoxyverbindung gemäß Anspruch 13, wobei Ar² eine 1-Cyclohexen-1,4-diylgruppe ist.

15. Das Verfahren zur Herstellung einer Epoxyverbindung gemäß einem der Ansprüche 1 bis 3, wobei Ar¹ und Ar³ unabhängig einen Rest der folgenden Formel bedeuten: 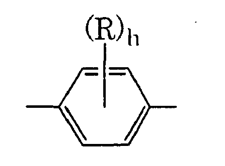 wobei R und h wie oben definiert sind und Ar² einen Rest der folgenden Formel bedeutet: 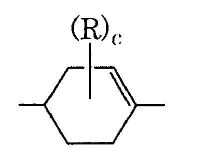 wobei R und c wie oben definiert sind.

16. Das Verfahren zur Herstellung einer Epoxyverbindung gemäß Anspruch 15, wobei Ar¹ und Ar³ unabhängig eine 1,4-Phenylengruppe, eine 3-Methyl-1,4-phenylengruppe oder eine 3-Isopropyl-1,4-phenylengruppe bedeuten und Ar² eine 1-Cyclohexen-1,4-diylgruppe bedeutet.

## Revendications

1. Procédé de production d'un composé époxy représenté par la formule (3) : 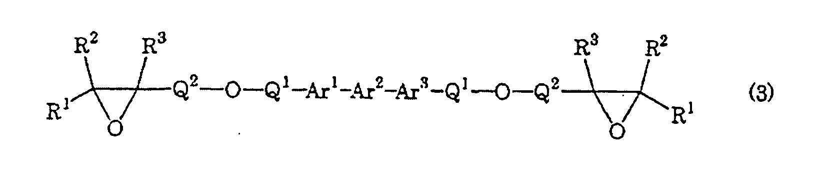 dans laquelle Ar¹, Ar², Ar³, R¹, R², R³, Q¹ et Q² sont comme définis ci-dessous, lequel comprend la réaction d'un composé dihydroxy représenté par la formule (1) :
HO-Q¹-Ar¹-Ar²-Ar³-Q¹-OH (1)
dans laquelle,
Q¹ représente une liaison simple ou un groupe alkylène à chaîne linéaire ayant de 1 à 9 atomes de carbone, dans laquelle au moins un groupe méthylène constituant le groupe alkylène à chaîne linéaire est éventuellement substitué avec un groupe alkyle ayant de 1 à 8 atomes de carbone ;
Ar¹, Ar² et Ar³ sont identiques ou différents et indiquent chacun l'un parmi des groupes divalents représentés par les formules suivantes : 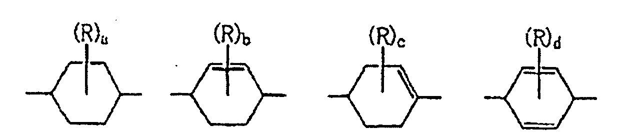 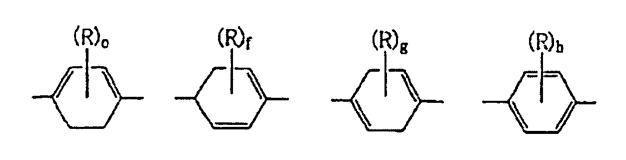 dans lesquelles R représente un groupe alkyle ayant de 1 à 8 atomes de carbone, a représente un nombre entier de 0 à 8, b, e et g représentent un nombre entier de 0 à 6, c représente un nombre entier de 0 à 7, d et h représentent un nombre entier de 0 à 4, f représente un nombre entier de 0 à 5, et lorsque plus d'un R existe dans ledit groupe divalent, tous les R peuvent être des groupes identiques ou des groupes différents ; et d'un composé représenté par la formule (2) : 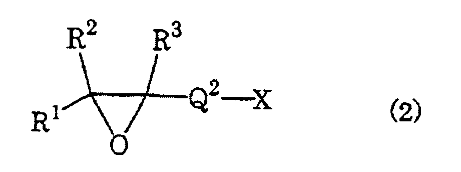 dans laquelle R¹, R² et R³ sont identiques ou différents et représentent chacun un atome d'hydrogène ou un groupe alkyle ayant de 1 à 8 atomes de carbone, Q² représente un groupe alkylène à chaîne linéaire ayant de 1 à 8 atomes de carbone, dans lequel au moins un groupe méthylène constituant le groupe alkylène à chaîne linéaire est éventuellement substitué avec un groupe alkyle ayant de 1 à 8 atomes de carbone, et X représente un atome d'halogène, en présence d'au moins un choisi dans le groupe constitué d'un composé d'amine et d'un sel d'ammonium, et de plus la réaction du mélange réactionnel obtenu et d'une base inorganique par mélange,
dans lequel la base inorganique est ajoutée au mélange réactionnel après que le composé dihydroxy représenté par la formule (1) et le composé représenté par la formule (2) ont réagi et que le composé dihydroxy représenté par la formule (1) a disparu et que 50 % ou plus d'un composé obtenu par réaction d'une molécule du composé dihydroxy représenté par la formule (1) et d'une molécule du composé représenté par la formule (2) ont disparu.

2. Procédé de production d'un composé époxy selon la revendication 1, dans lequel une base inorganique est ajoutée au mélange réactionnel après que le composé dihydroxy représenté par la formule (1) a disparu et que 80 % ou plus d'un composé obtenu par réaction d'une molécule du composé dihydroxy représenté par la formule (1) et d'une molécule du composé représenté par la formule (2) ont disparu.

3. Procédé de production d'un composé époxy selon la revendication 1, dans lequel une base inorganique est ajoutée au mélange réactionnel après que le composé dihydroxy représenté par la formule (1) a disparu et que 90 % ou plus d'un composé obtenu par réaction d'une molécule du composé dihydroxy représenté par la formule (1) et d'une molécule du composé représenté par la formule (2) ont disparu.

4. Procédé de production d'un composé époxy selon l'une quelconque des revendications 1 à 3, dans lequel le composé dihydroxy représenté par la formule (1) et le composé représenté par la formule (2) réagissent en présence d'un sel d'ammonium.

5. Procédé de production d'un composé époxy selon l'une quelconque des revendications 1 à 3, dans lequel le composé d'amine est une amine tertiaire.

6. Procédé de production d'un composé époxy selon la revendication 5, dans lequel l'amine tertiaire est la triéthylamine.

7. Procédé de production d'un composé époxy selon l'une quelconque des revendications 1 à 3, dans lequel le sel d'ammonium est un sel d'ammonium quaternaire.

8. Procédé de production d'un composé époxy selon la revendication 7, dans lequel le sel d'ammonium quaternaire est un halogénure d'ammonium quaternaire.

9. Procédé de production d'un composé époxy selon la revendication 8, dans lequel l'halogénure d'ammonium quaternaire est le chlorure de trétraalkylammonium ou le bromure de tétraalkylammonium.

10. Procédé de production d'un composé époxy selon l'une quelconque des revendications 1 à 3, dans lequel la base inorganique est un hydroxyde de métal alcalin.

11. Procédé de production d'un composé époxy selon l'une quelconque des revendications 1 à 3, dans lequel Ar¹ et Ar³ représentent indépendamment un groupe représenté par la formule suivante : 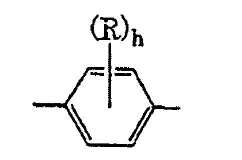 dans laquelle R et h sont comme définis ci-dessus.

12. Procédé de production d'un composé époxy selon la revendication 11, dans lequel Ar¹ et Ar³ représentent indépendamment un groupe 1,4-phénylène, un groupe 3-méthyl-1,4-phénylène ou un groupe 3-isopropyl-1,4-phénylène.

13. Procédé de production d'un composé époxy selon l'une quelconque des revendications 1 à 3, dans lequel Ar² est un groupe représenté par la formule suivante : 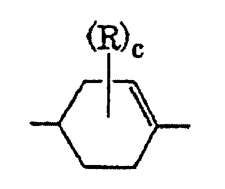 dans laquelle R et c sont comme définis ci-dessus.

14. Procédé de production d'un composé époxy selon la revendication 13, dans lequel Ar² est un groupe 1-cyclohexène-1,4-diyle.

15. Procédé de production d'un composé époxy selon l'une quelconque des revendications 1 à 3, dans lequel Ar¹ et Ar³ représentent indépendamment un groupe représenté par la formule suivante : 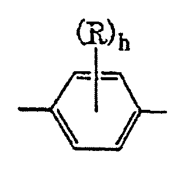 dans laquelle R et h sont comme définis ci-dessus, et Ar² représente un groupe représenté par la formule suivante : 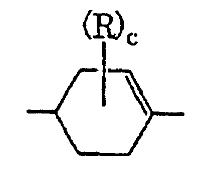 dans laquelle R et c sont comme définis ci-dessus.

16. Procédé de production d'un composé époxy selon la revendication 15, dans lequel Ar¹ et Ar³ représentent indépendamment un groupe 1,4-phénylène, un groupe 3-méthyl-1,4-phénylène ou un groupe 3-isopropyl-1,4-phénylène, et Ar² représente un groupe 1-cyclohexène-l,4-diyle.
